# EUROPÄISCHE PATENTSCHRIFT

(11) **EP 0 557 860 B1**
(45) Veröffentlichungstag und Bekanntmachung des Hinweises auf die Patenterteilung: **10.01.2001**
(21) Anmeldenummer: 93102388.1
(22) Anmeldetag: 16.02.1993
(51) Int. Cl.: C07D 401/04, A01N 43/54

(54) **Substituierte Pyridylpyrimidine und ihre Verwendung als Schädlingsbekämpfungsmittel**
Substituted pyridiylpyrimidines and their use as parasiticide
Pyridylpyrimidines substituées et leur utilisation comme parasiticide

(30) Priorität: 28.02.1992 DE 4206148
(43) Veröffentlichungstag der Anmeldung: 01.09.1993
(73) Patentinhaber: BAYER AG, 51368 Leverkusen (DE)
(72) Erfinder: Heinemann, Ulrich, Dr., W-5653 Leichlingen 2 (DE); Himmler, Thomas, Dr., W-5068 Odenthal (DE); Dutzmann, Stefan, Dr., W-4010 Hilden (DE); Hänssler, Gerd, Dr., W-5090 Leverkusen 3 (DE); Dehne, Heinz-Wilhelm, Dr. habil., W-4019 Monheim (DE)

(56) Entgegenhaltungen:
- EP-A- 0 407 899
- DE-B- 1 271 116

## Beschreibung

Die Erfindung betrifft neue substituierte Pyridylpyrimidine, ein Verfahren zu ihrer Herstellung und ihre Verwendung zur Bekämpfung von phytopathogenen Pilzen.

Es ist bekannt, daß bestimmte substituierte Pyridylpyrimidine wie beispielsweise die Verbindung 2-(2-Pyridyl)-4-n-propylamino-6-phenyl-pyrimidin fungizide Eigenschaften besitzen (vergleiche EP-A 0 407 899).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Weiterhin geht aus der DE-B 1 271 116 hervor, daß 4-Hydroxypyrimidine, die auch in der 6-Position einen heterocyclischen Rest enthalten, pharmakologische Eigenschaften besitzen und gegen Bakterien sowie humanpathogene Pilze wirksam sind. Ein Einsatz dieser Stoffe gegen phytopathogene Pilze wird jedoch nicht erwähnt.

Es wurden neue substituierte Pyridylpyrimidine der Formel 1 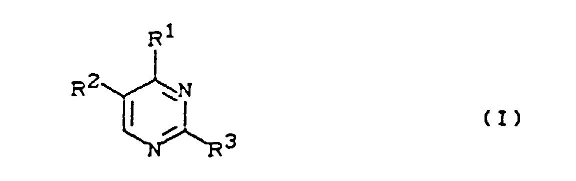 in welcher
- R¹: für geradkettiges oder verzweigtes Alkyl mit 1 bis 4 Kohlenstoffatomen, für geradkettiges oder verzweigtes Halogenalkyl mit 1 bis 4-Kohlenstoffatomen und 1 bis 9 gleichen oder verschiedenen Halogenatomen, oder für gegebenenfalls einfach bis vierfach, gleich oder verschieden substituiertes Cycloalkyl mit 3 bis 6 Kohlenstoffatomen steht, wobei als Substituenten infrage kommen:
Halogen, geradkettiges oder verzweigtes Alkyl mit 1 bis 4 Kohlenstoffatomen oder geradkettiges oder verzweigtes Halogenalkyl mit 1 bis 4 Kohlenstoffatomen und 1 bis 9 gleichen oder verschiedenen Halogenatomen,
- R²: für gegebenenfalls einfach bis fünffach, gleich oder verschieden substituiertes Phenyl steht, wobei als Substituenten jeweils infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Trifluormethyl, Trifluormethoxy, Trifluormethylthio, Methoxycarbonyl, Ethoxycarbonyl, Methoximinomethyl, Methoximinoethyl, Ethoximinomethyl, Ethoximinoethyl oder gegebenenfalls ein- bis dreifach, gleich oder verschieden durch Fluor, Chlor, Brom, Methyl und/oder Ethyl substituiertes Phenyl, und
- R³: für gegebenenfalls einfach bis vierfach, gleich oder verschieden substituiertes 2-Pyridyl oder 4-Pyridyl steht, wobei als Substituenten jeweils infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Trifluormethyl, Trifluormethoxy, Trifluormethylthio, Methoxycarbonyl, Ethoxycarbonyl, Methoximinomethyl, Methoximinoethyl, Ethoximinomethyl, Ethoximinoethyl oder gegebenenfalls ein- bis dreifach, gleich oder verschieden durch Fluor, Chlor, Brom, Methyl und/oder Ethyl substituiertes Phenyl,
gefunden.

Weiterhin wurde gefunden, daß man die neuen substituierten Pyridylpyrimidine der Formel (I) erhält, wenn man Pyridylamidin Hydrochloride der Formel 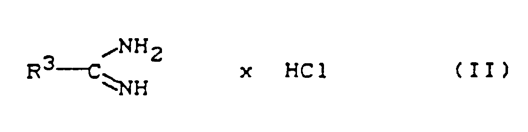 in welcher
- R³: die oben angegebene Bedeutung hat,
mit Enaminoketonen der Formel 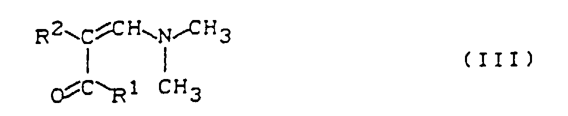 in welcher
- R¹ und R²: die oben angegebene Bedeutung haben, gegebenenfalls in Gegenwart eines Verdünnungsmittels und gegebenenfalls in Gegenwart eines Säurebindemittels umsetzt.

Schließlich wurde gefunden, daß die neuen substituierten Pyridylpyrimidine der Formel (I) eine gute fungizide Wirksamkeit gegen phytopathogene Pilze besitzen.

Überraschenderweise zeigen die erfindungsgemäßen substituierten Pyridylpyrimidine der allgemeinen Formel (I) eine deutlich bessere fungizide Wirksamkeit gegen phytopathogene Pilze als die aus dem Stand der Technik bekannten substituierten Pyridylpyrimidine, wie beispielsweise die Verbindung 2-(2-Pyridyl)-4-n-propylamino-6-phenyl-pyrimidin, welches chemisch und wirkungsmäßig naheliegende Verbindungen sind.

Die erfindungsgemäßen substituierten Pyridylpyrimidine sind durch die Formel (I) allgemein definiert. Bevorzugt sind Verbindungen der Formel (I), bei welchen
- R¹: Methyl, Ethyl, n- oder i-Propyl, n-, i-, s- oder t-Butyl, für geradkettiges oder verzweigtes Halogenalkyl mit 1 bis 4 Kohlenstoffatomen und 1 bis 5 gleichen oder verschiedenen Halogenatomen - insbesondere Fluor oder Chlor - oder für jeweils gegebenenfalls einfach oder zweifach, gleich oder verschieden substituiertes Cyclopropyl, Cyclobutyl, Cyclopentyl oder Cyclohexyl steht, wobei als Cycloalkylsubstituenten jeweils infrage kommen:
Fluor, Chlor, Brom, Methyl, Ethyl, n- oder i-Propyl, Chlormethyl oder Trifluormethyl,
- R²: für gegebenenfalls einfach bis dreifach, gleich oder verschieden substituiertes Phenyl steht, wobei als Substituenten jeweils infrage kommen:
Fluor, Chlor, Brom, Cyano, Nitro, Methyl, Ethyl,
n- oder i-Propyl, n-, i-, s- oder t-Butyl, Methoxy, Ethoxy, n- oder i-Propoxy, n-, i-, s- oder t-Butoxy, Methylthio, Ethylthio, Trifluormethyl, Trifluormethoxy oder Trifluormethylthio und
- R³: für gegebenenfalls einfach oder zweifach, gleich oder verschieden substituiertes 2-Pyridyl steht, wobei als Substituenten jeweils die bei R² genannten infrage kommen.

Im einzelnen seien außer den bei den Herstellungsbeispielen genannten Verbindungen die folgenden substituierten Pyridylpyrimidine der Formel (I) genannt:

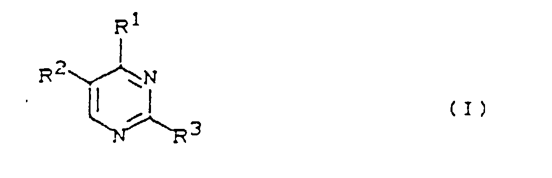

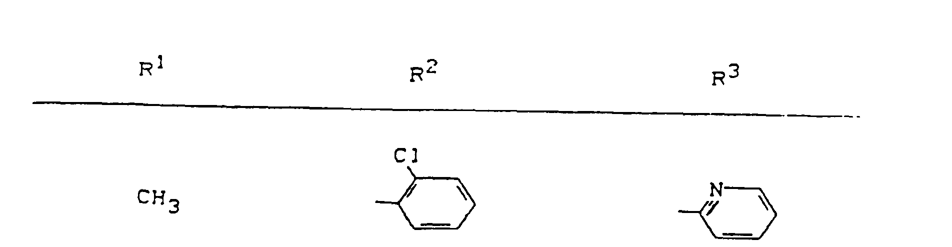

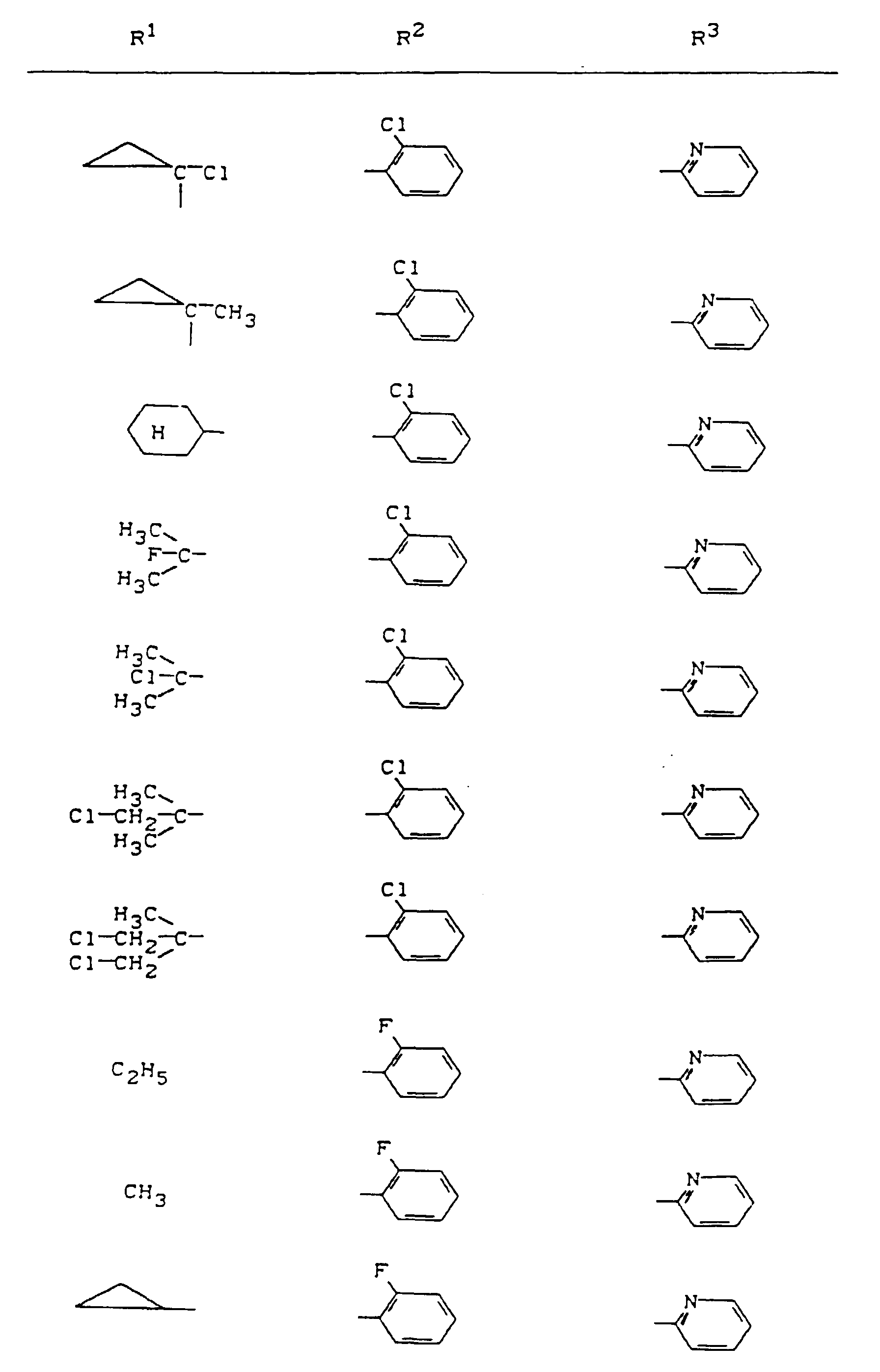

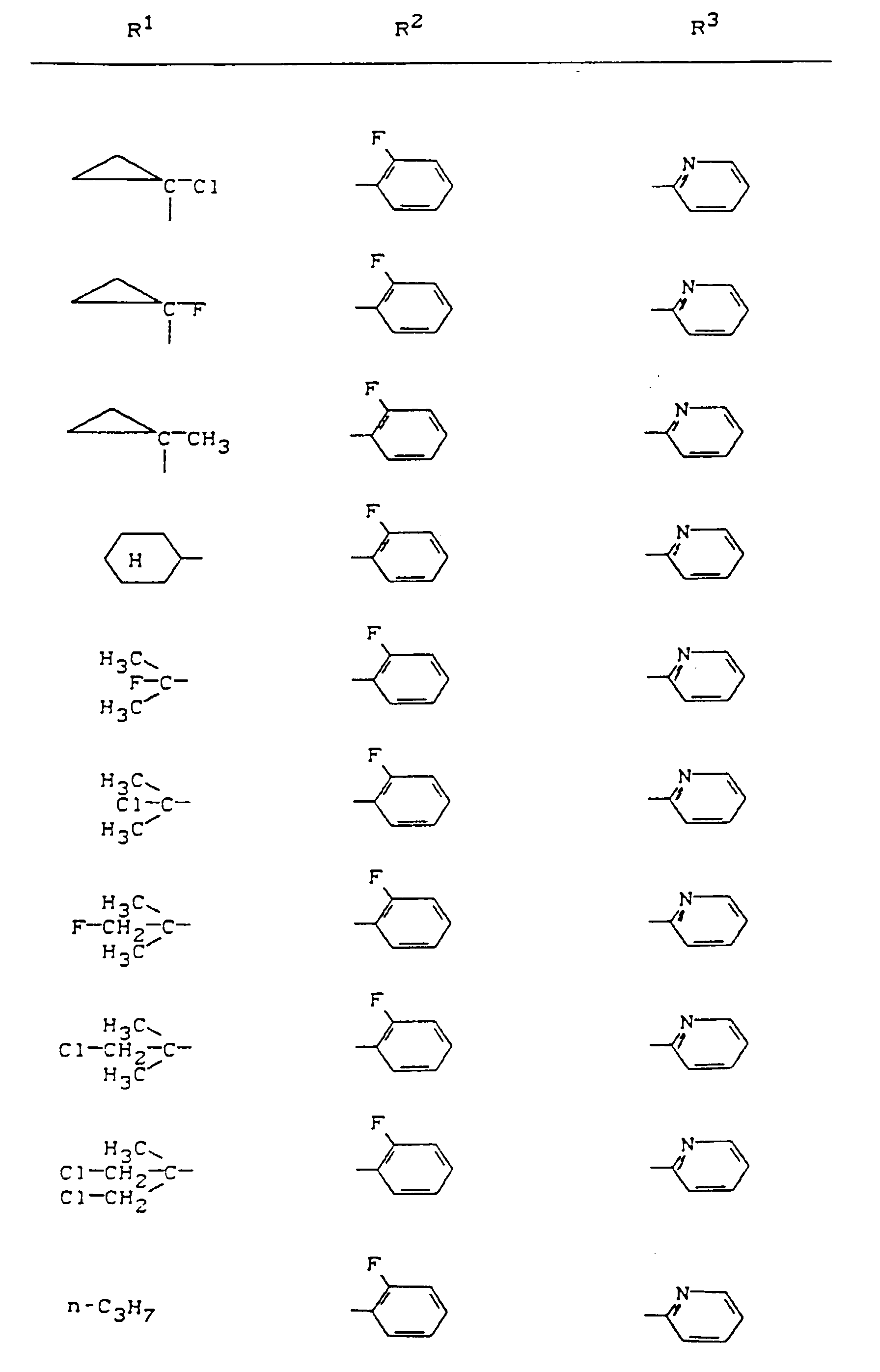

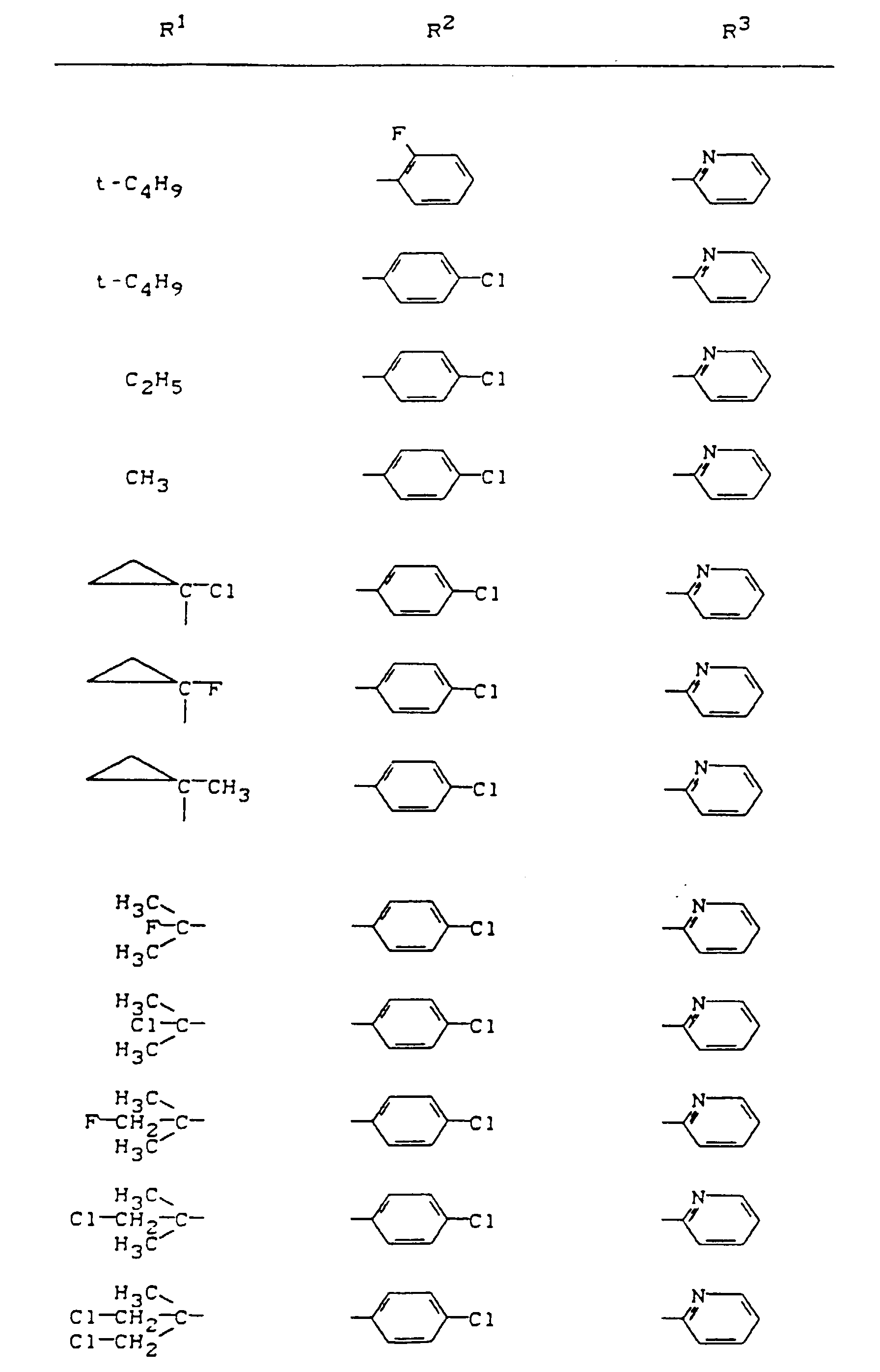

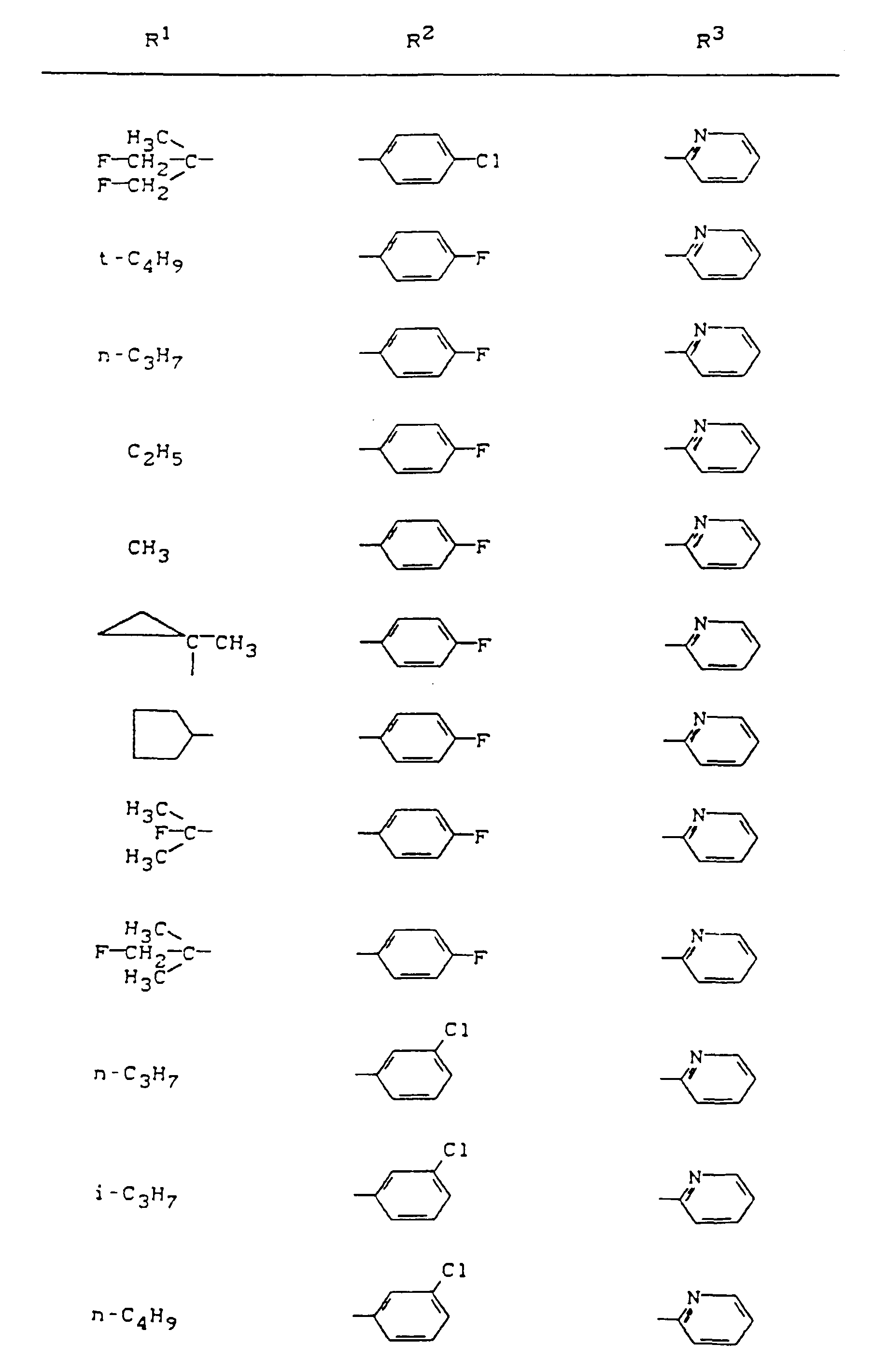

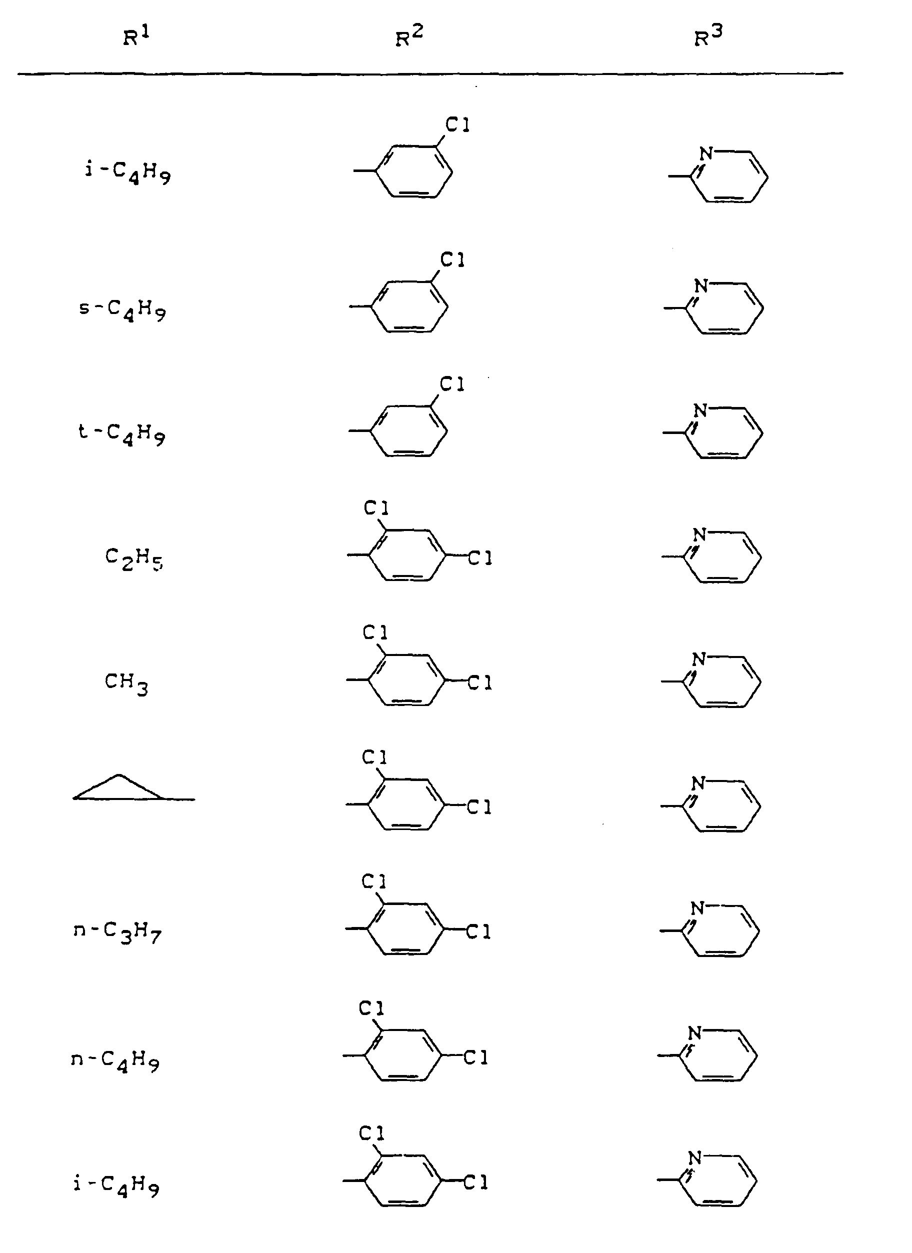

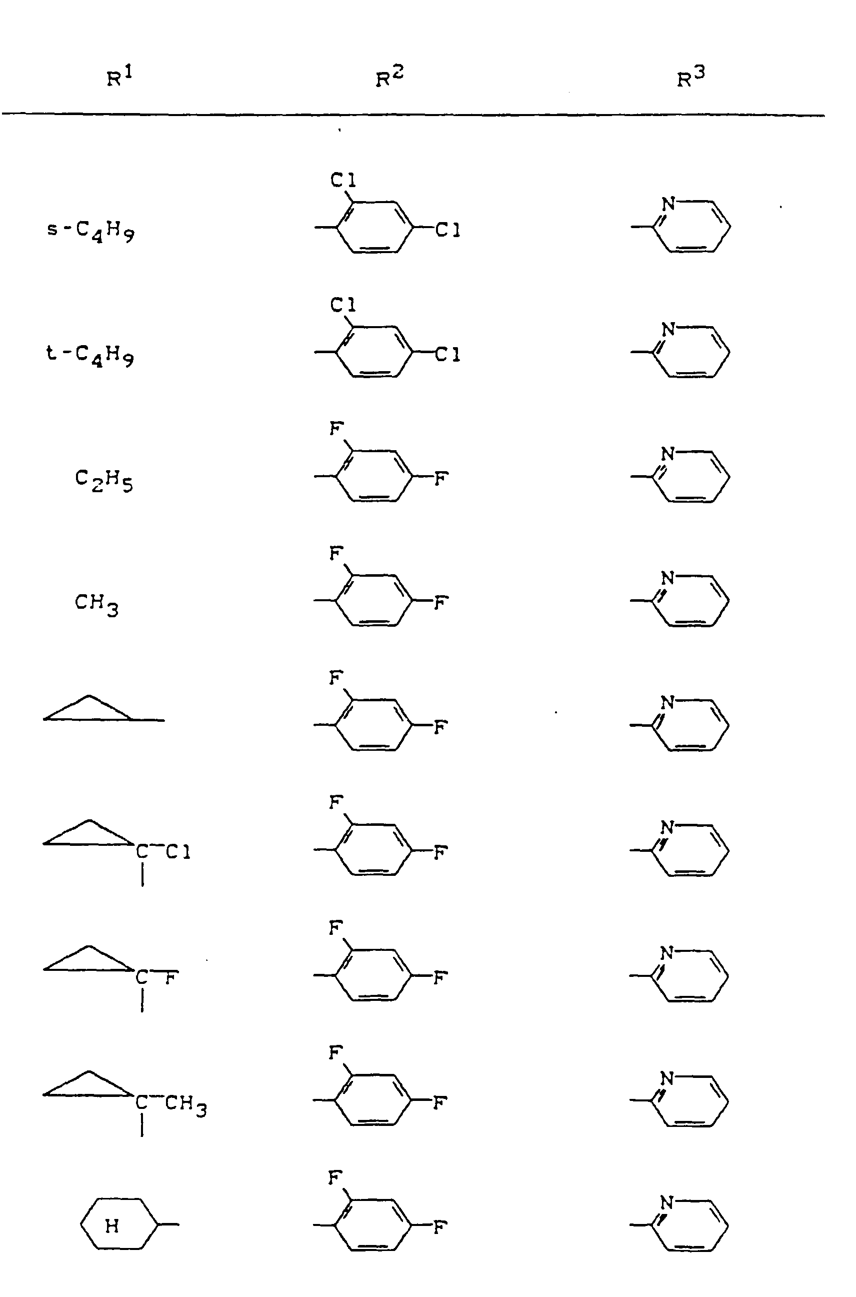

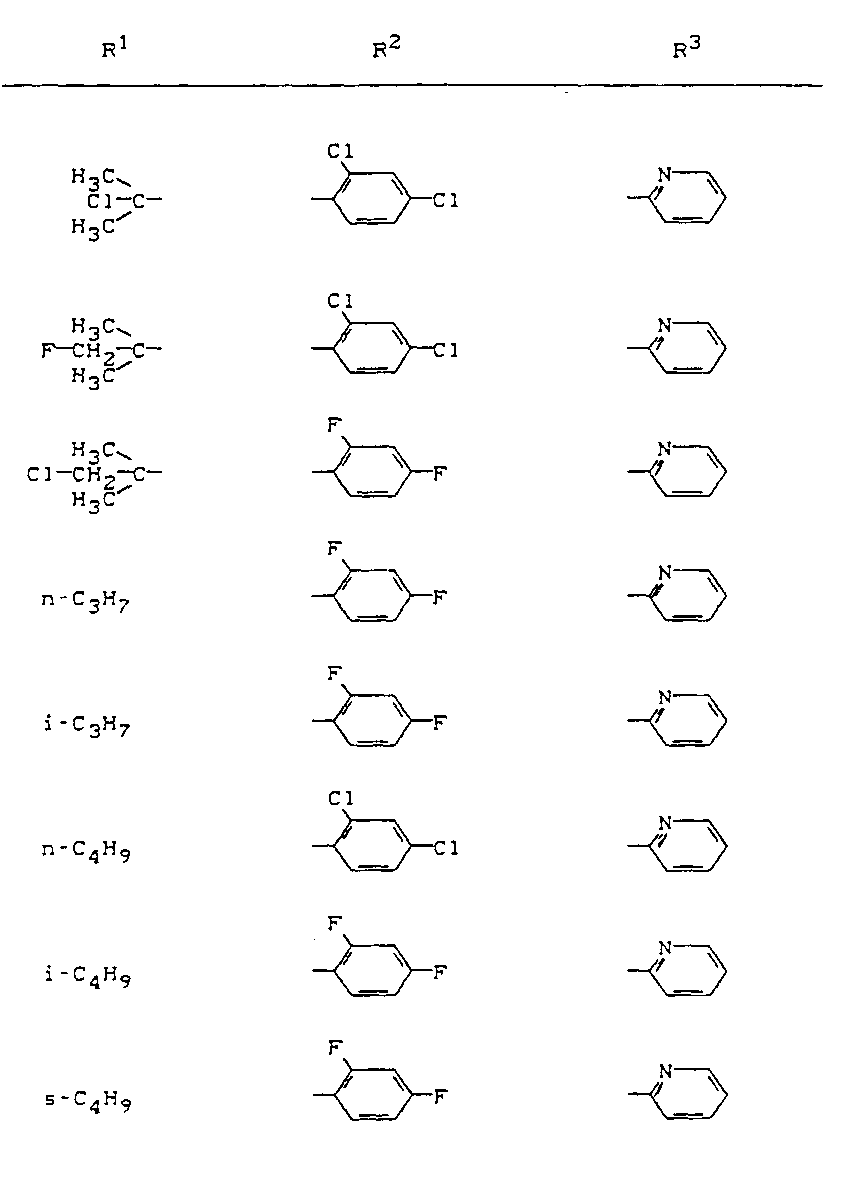

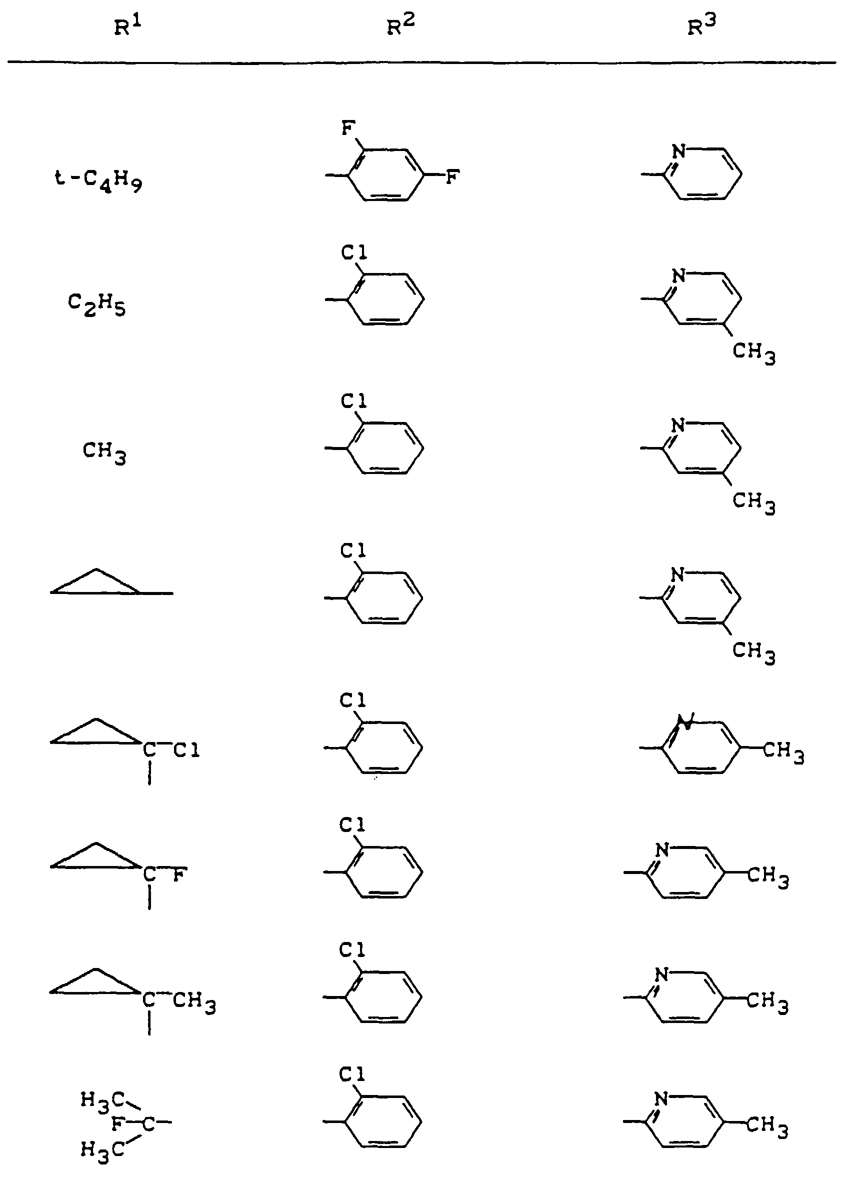

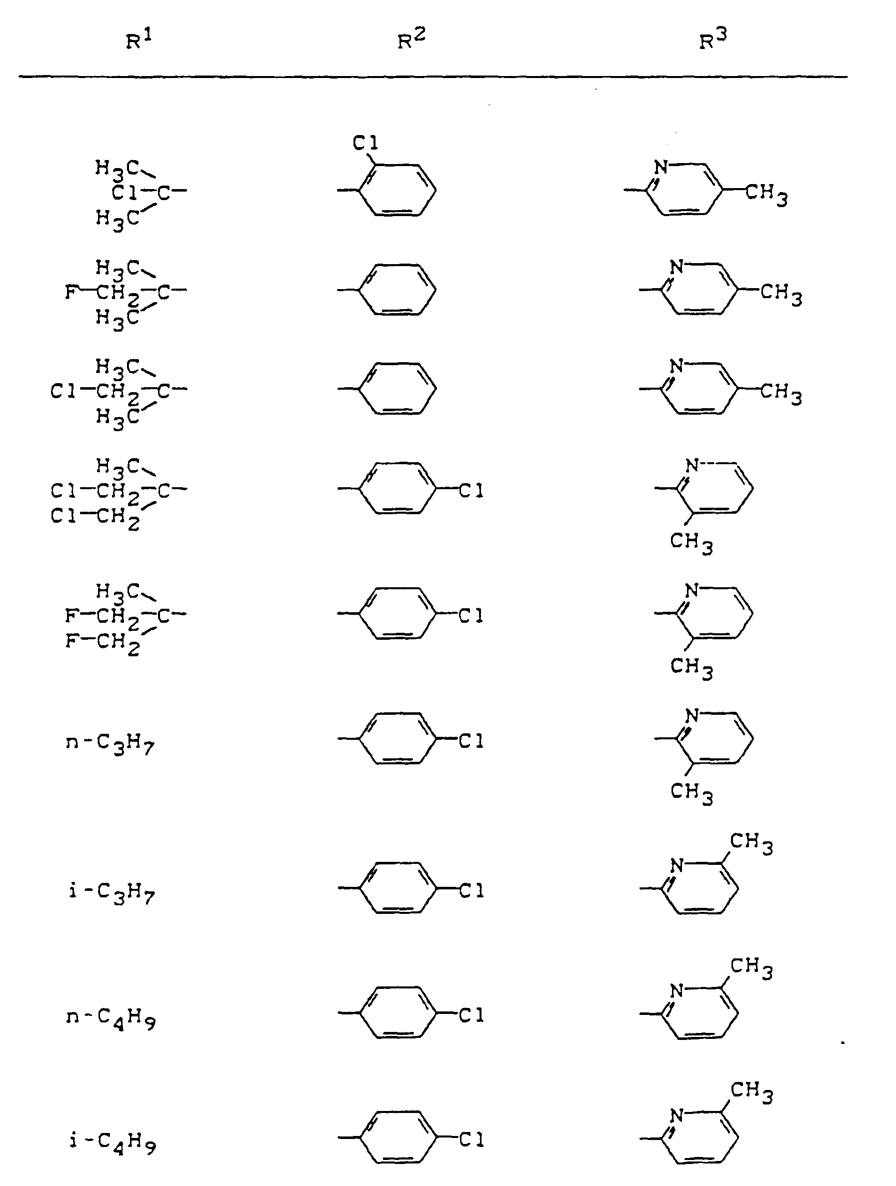

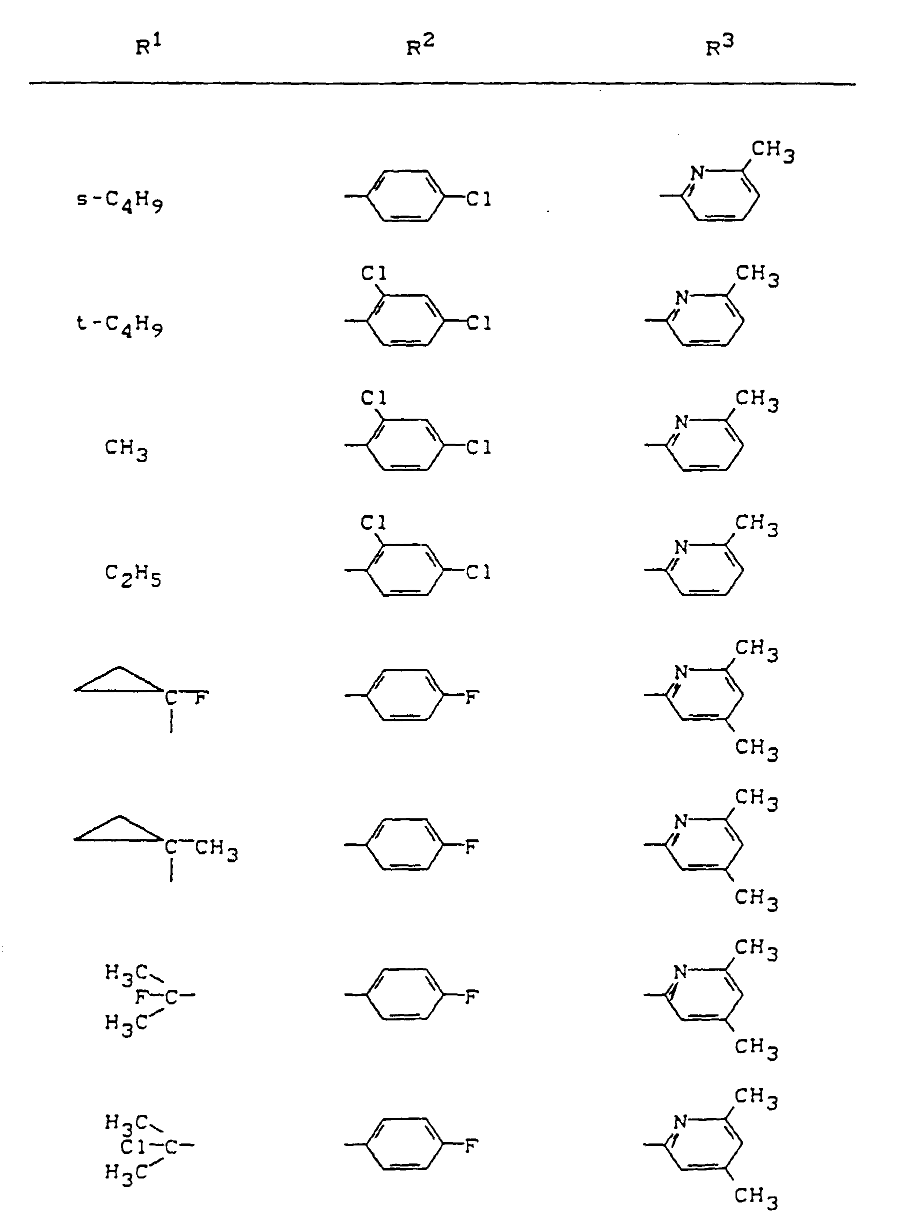

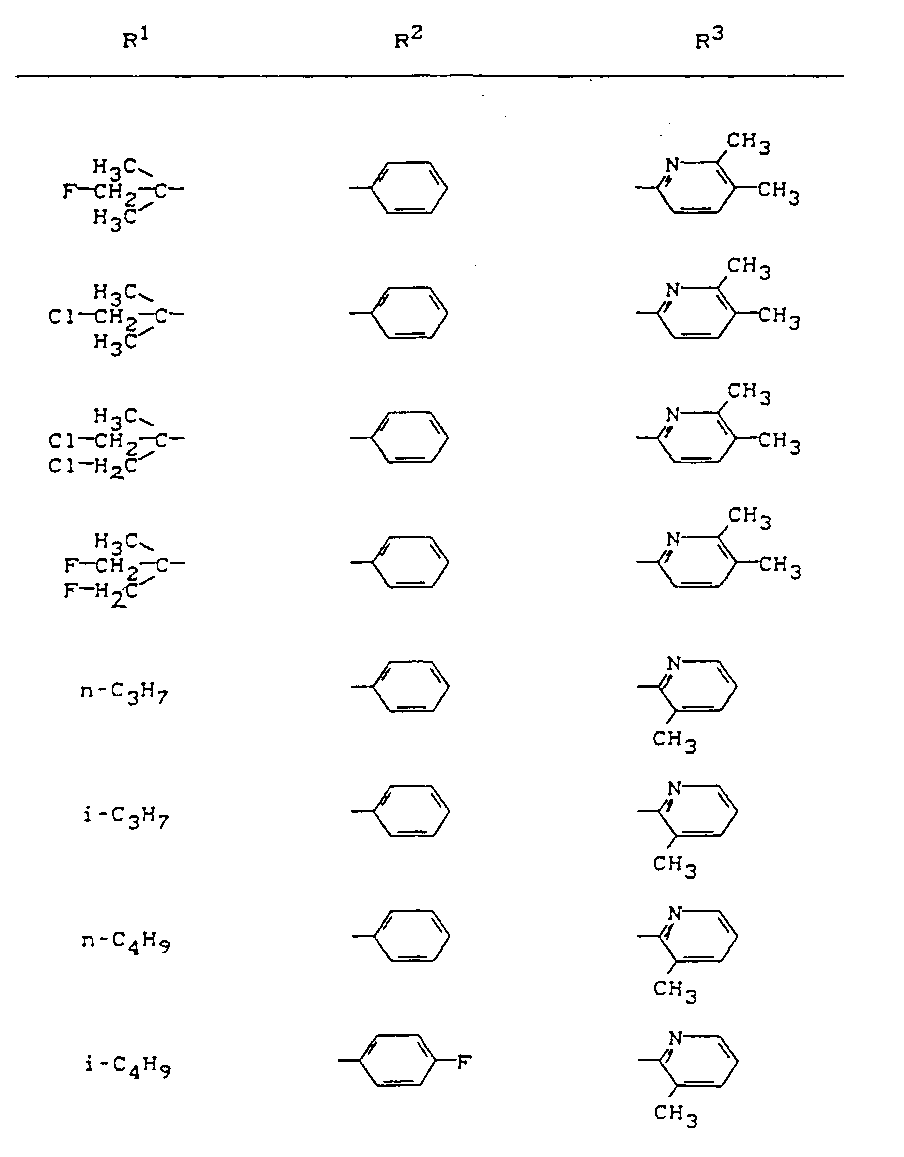

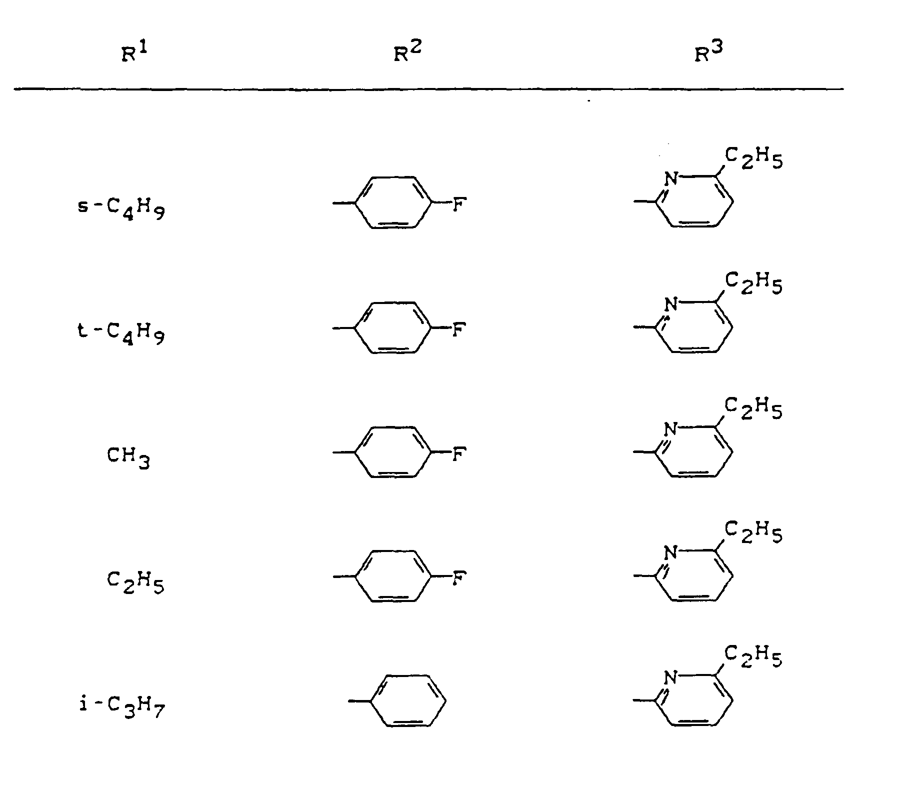

Verwendet man beispielsweise 2-Pyridylamidin Hydrochlorid und 4-Dimethylamino-3-phenyl-but-3-en-2-on als Ausgangsverbindungen, so läßt sich der Reaktionsablauf des erfindungsgemäßen Verfahrens durch das folgende Formelschema darstellen:

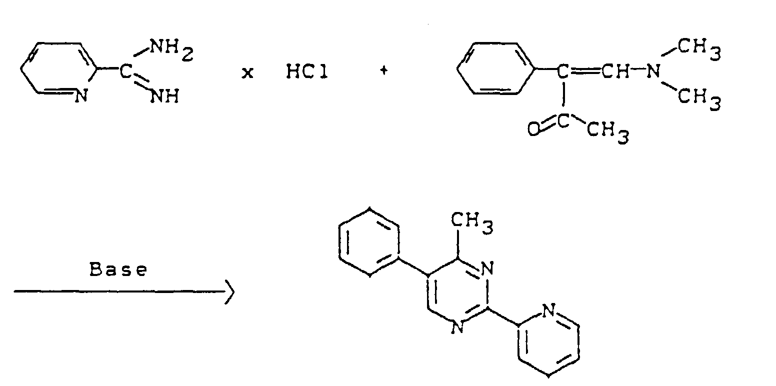

Die zur Durchführung des erfindungsgemäßen Verfahrens als Ausgangsverbindungen benötigten Pyridylamidin Hydrochloride sind durch die Formel (II) allgemein definiert. In dieser Formel (II) steht R³ vorzugsweise für diejenigen Reste, die bereits im Zusammenhang mit der Beschreibung der erfindungsgemäßen Verbindungen der Formel (I) als bevorzugt für diesen Substituenten genannt wurden. Die Pyridylamidin Hydrochloride der Formel (II) sind bekannt oder erhältlich in Analogie zu bekannten Verfahren (vergleiche EP-A 0 259 139 oder EP-A 0 270 362).

Die zur Durchführung des erfindungsgemäßen Verfahrens weiterhin als Ausgangsstoffe benötigten Enaminoketone sind durch die Formel (III) allgemein definiert. In dieser Formel (III) stehen R¹ und R² vorzugsweise für diejenigen Reste, die bereits im Zusammenhang mit der Beschreibung der erfindungsgemäßen Verbindungen der Formel (I) als bevorzugt für diese Substituenten genannt wurden.
Die Enaminoketone der Formel (III) sind bekannt oder erhältlich in Analogie zu bekannten Verfahren (vergleiche z.B. DE-A 33 15 797; EP-A 102 227; DE-A 33 17 289; EP-A 61 774; DE-A 30 12 597; FR-A 24 77 148; J. Org. Chem. 44, 835-839 [1979]; J. Med. Chem. 21, 623-628 [1978]).

Als Verdünnungsmittel zur Durchführung des erfindungsgemäßen Verfahrens kommen inerte organische Lösungsmittel infrage. Hierzu gehören insbesondere aliphatische, alicyclische oder aromatische, gegebenenfalls halogenierte Kohlenwasserstoffe wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff; Ether wie Diethylether, Diisopropylether, Dioxan, Tetrahydrofuran oder Ethylenglykoldimethyl- oder -diethylether; Ketone wie Aceton oder Butanon oder Methyl-isobutyl-keton; Nitrile wie Acetonitril, Propionitril oder Benzonitril; Amide wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester oder Sulfoxide wie Dimethylsulfoxid oder Alkohole wie Methanol, Ethanol sowie n- oder i-Propanol.

Das erfindungsgemäße Verfahren wird vorzugsweise in Gegenwart eines geeigneten Reaktionshilfsmittels durchgeführt. Als solche kommen alle üblichen anorganischen und organischen Basen infrage. Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-t-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens in einem größeren Bereich variiert werden. Im allgemeinen arbeitet man bei Temperaturen zwischen 20°C und 140°C, vorzugsweise bei Temperaturen zwischen 60°C und 120°C.

Das erfindungsgemäße Verfahren wird üblicherweise unter Normaldruck durchgeführt. Es ist jedoch auch möglich unter erhöhtem oder vermindertem Druck zu arbeiten.

Zur Durchführung des erfindungsgemäßen Verfahrens setzt man pro Mol an Pyridylamidin Hydrochlorid der Formel (II) im allgemeinen 0.8 bis 1.5 Mol, vorzugsweise 1.0 bis 1.2 Mol an Enaminoketon der Formel (III) und gegebenenfalls 1.0 bis 2.0 Mol, vorzugsweise 1.0 bis 1.5 Mol an als Reaktionshilfsmittel verwendeter Base ein.

Die Reaktionsdurchführung, Aufarbeitung und Isolierung der Reaktionsprodukte erfolgt nach bekannten Verfahren (vergleiche hierzu auch die Herstellungsbeispiele).

Die erfindungsgemäßen Wirkstoffe weisen eine starke Wirkung gegen phytopathogene Pilze auf und können zur Bekämpfung von derartigen unerwünschten Schadorganismen praktisch eingesetzt werden. Die Wirkstoffe sind für den Gebrauch als Pflanzenschutzmittel, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Krankheiten, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Dabei können die erfindungsgemäßen Wirkstoffe mit besonders gutem Erfolg zur Bekämpfung von Getreidekrankheiten, wie beispielsweise gegen den Erreger der Braunspelzigkeit des Weizens (Leptosphaeria nodorum) oder gegen den Erreger des echten Getreidemehltaues an Weizen oder Gerste (Erysiphe graminis) oder gegen Krankheiten im Obst- und Gemüseanbau, wie beispielsweise gegen den Erreger des Rebenmehltaues (Uncinula necator) oder gegen den Erreger des Apfelmehltaues (Podosphaera leucotricha) oder gegen den Erreger des Gurkenmehltaues (Sphaerotheca fuliginea) oder zur Bekämpfung von Krankheiten im Reisanbau wie beispielsweise gegen den Erreger der Reisfleckenkrankheit (Pyricularia oryzae) oder gegen den Erreger der Reisstengelkrankheit (Pellicularia sasakii) eingesetzt werden. Daneben besitzen die erfindungsgemäßen Wirkstoffe auch eine gute in vitro-Wirksamkeit.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.
Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 Gew.-% am Wirkungsort erforderlich.

### Herstellungsbeispiele

### Beispiel 1

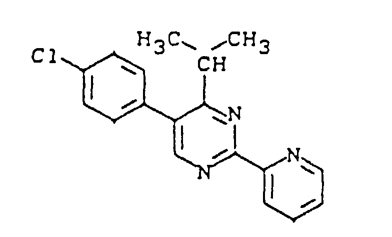

3,2 g (0,02 Mol) Pyridin-2-carbamidin Hydrochlorid (vergleiche z.B. EP 270 362), 5,0 g (0,02 Mol) 1-Dimethylamino-2-(4-chlorphenyl)-4-methyl-pent-1-en-3-on und 1,3 g (0,024 Mol) Natriummethylat werden in 50 ml trockenem Methanol für 4 Stunden auf Rückflußtemperatur erhitzt, anschließend auf Raumtemperatur abgekühlt, mit Eisessig neutralisiert, im Vakuum eingeengt, der Rückstand in t-Butyl-methyl-ether aufgenommen, mit Wasser gewaschen, über Natriumsulfat getrocknet, abermals im Vakuum eingeengt, der kristalline Rückstand mit Diethylether verrührt, abgesaugt und getrocknet.

Man erhält 4,0 g (65 % der Theorie) an 2-(2-Pyridyl)-5-(4-chlorphenyl)-4-isopropyl-pyrimidin vom Schmelzpunkt 145°C.

### Herstellung der Ausgangsverbindung

### Beispiel III-1

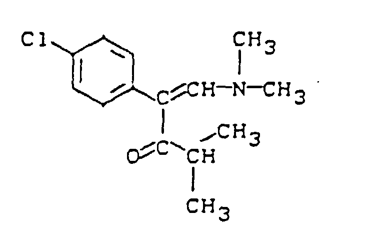

8,5 g (0,041 Mol) 3-Methyl-1-(4-chlorphenyl)-butan-2-on (Herstellung vergleiche z.B. EP 461 483) und 5,9 g (0,049 Mol) N,N-Dimethylformamid-dimethylacetal werden für 16 Stunden auf Rückflußtemperatur erhitzt, anschließend auf Raumtemperatur abgekühlt, im Vakuum eingeengt und der kristalline Rückstand aus Ligroin umkristallisiert.

Man erhält 9,0 g (87 % der Theorie) an 1-Dimethylamino-2-(4-chlorphenyl)-4-methyl-pent-1-en-3-on vom Schmelzpunkt 95°C.

In entsprechender Weise und gemäß den allgemeinen Angaben zur Herstellung erhält man die folgenden substituierten Pyridylpyrimidine der Formel

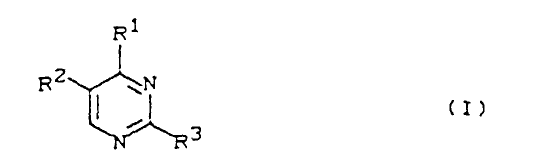

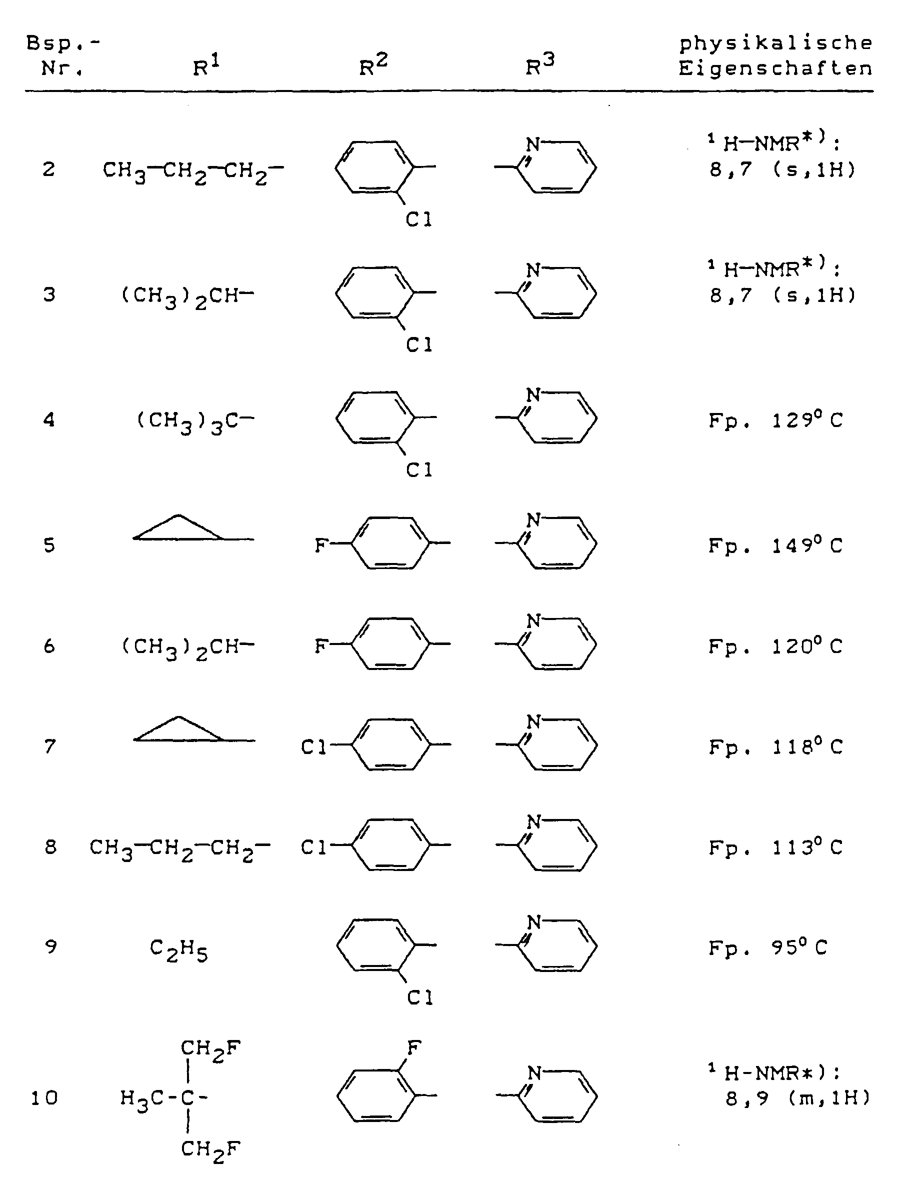

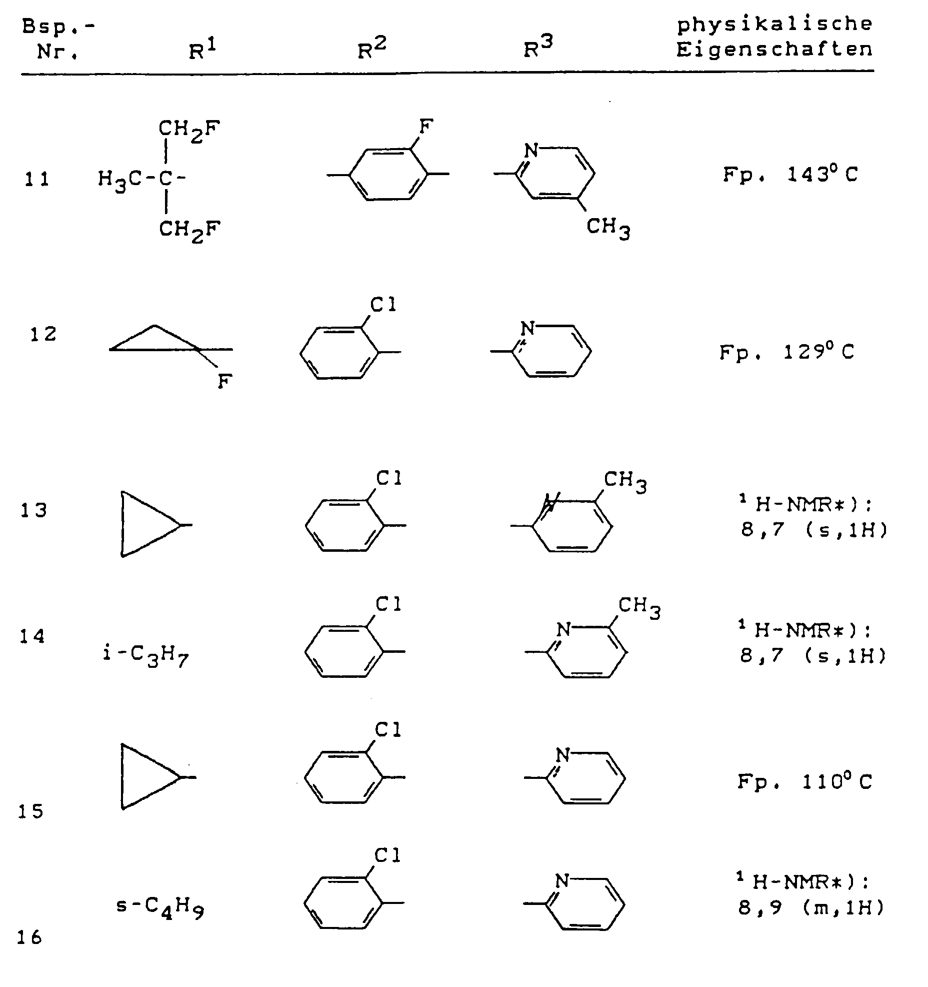

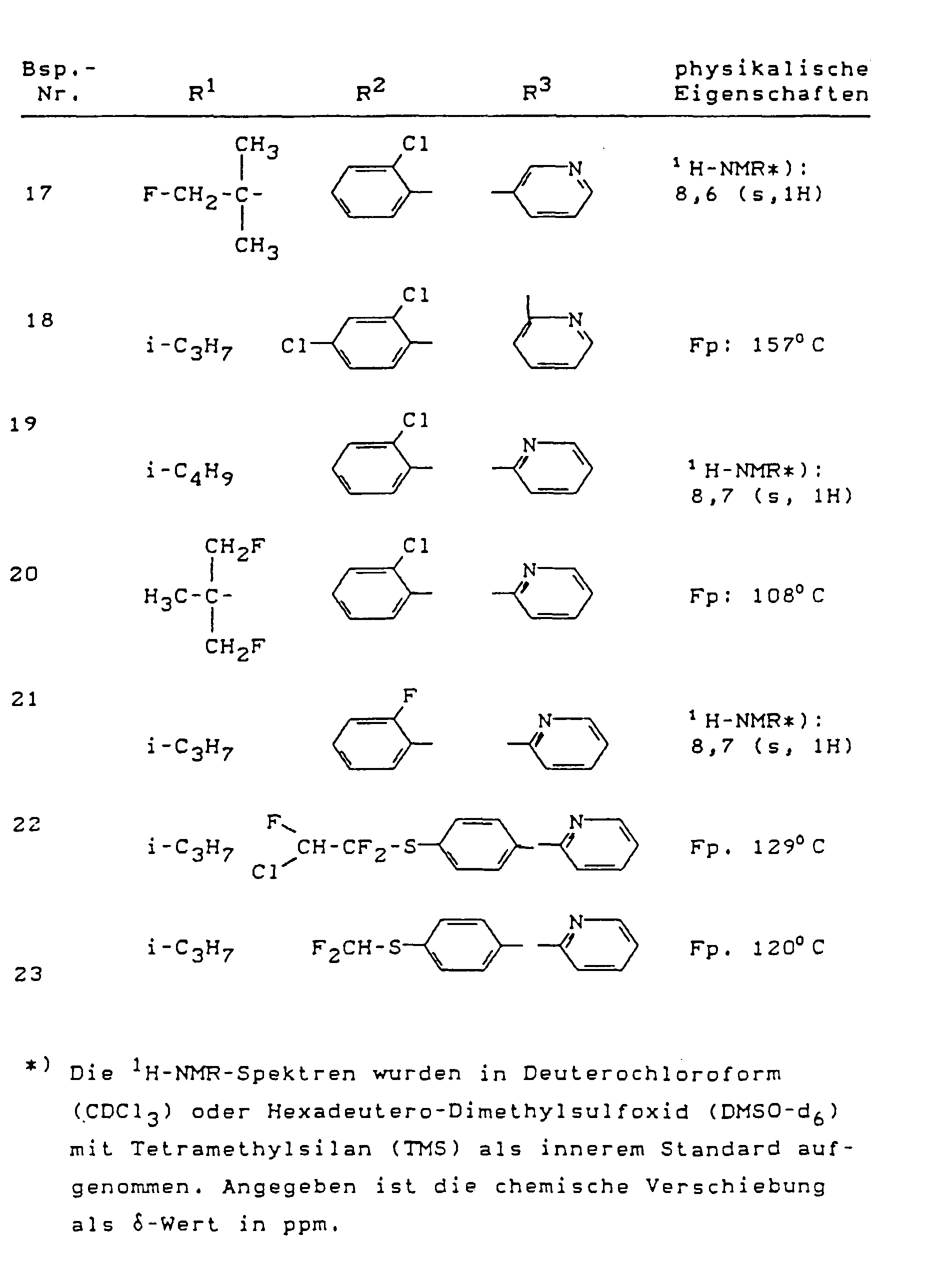

### Anwendungsbeispiele

In den folgenden Anwendungsbeispielen wurden die nachstehend aufgeführten Verbindungen als Vergleichssubstanzen eingesetzt: 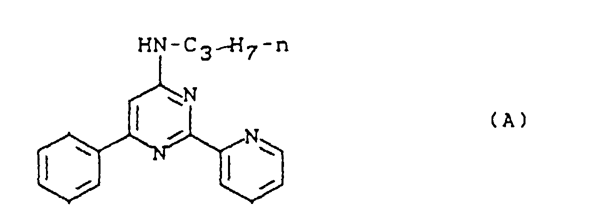 2-(2-Pyridyl)-4-n-propylamino-6-phenyl-pyrimidin (bekannt aus EP-A 407 899) 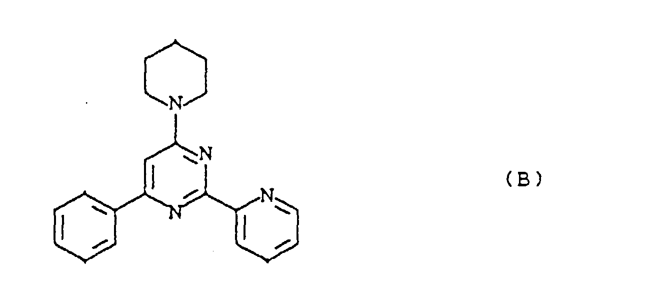 2-(2-Pyridyl)-4-(1-piperidinyl)-6-phenyl-pyrimidin (bekannt aus EP-A 407 899)

### Beispiel A

Leptosphaeria nodorum-Test (Weizen) / protektiv
Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Sporensuspension von Leptosphaeria nodorum besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 15°C und einer relativen Luftfeuchtigkeit von ca. 80 % aufgestellt.

10 Tage nach der Inokulation erfolgt die Auswertung.

Eine deutliche Überlegenheit in der Wirksamkeit gegenüber der Verbindung (A) aus dem Stand der Technik zeigen bei diesem Test z.B. die Verbindungen gemäß den Herstellungsbeispielen: 3 und 4.

### Beispiel B

Erysiphe-Test (Gerste) / protektiv
Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 25°C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber der Verbindung (A) aus dem Stand der Technik zeigt bei diesem Test z.B. die Verbindung gemäß Herstellungsbeispiel 3.

### Beispiel C

Erysiphe-Test (Weizen) / protektiv
Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.tritici bestäubt.

Die Pflanzen werden in einem Gewächshaus bei einer Temperatur von ca. 25 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber der Verbindung (A) aus dem Stand der Technik zeigt bei diesem Test z.B. die Verbindung gemäß Herstellungsbeispiel 3.

### Beispiel D

Uncinula-Test (Rebe) / protektiv
Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung bis zur Tropfnässe. Nach Antrocknen des Spritzbelages werden die Pflanzen mit Konidien des Pilzes Uncinula necator bestäubt.

Die Pflanzen werden in einem Gewächshaus bei einer Temperatur von 23°C bis 24°C und einer relativen Luftfeuchtigkeit von ca. 75 % aufgestellt.

14 Tage nach der Inokulation erfolgt die Auswertung.

Eine deutliche Überlegenheit in der Wirksamkeit gegenüber der Verbindung (A) aus dem Stand der Technik zeigt bei diesem Test z.B. die Verbindung gemäß Herstellungsbeispiel 4 u.a..

### Beispiel E

Pyricularia-Test (Reis) / protektiv
Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach Antrocknen des Spritzbelages werden die Pflanzen mit einer wäßrigen Sporensuspension von Pyricularia oryzae inokuliert.

Die Pflanzen werden anschließend im Gewächshaus bei 25°C und einer relativen Luftfeuchtigkeit von 100 % aufgestellt.

4 Tage nach der Inokulation erfolgt die Auswertung des Krankheitsbefalls.

Eine deutliche Überlegenheit in der Wirksamkeit gegenüber der Verbindung (B) aus dem Stand der Technik zeigen in diesem Test z.B. die Verbindungen gemäß den Herstellungsbeispielen: 1, 3, 4, 5, 6, 7 und 8.

### Beispiel F

Pellicularia-Test (Reis)
Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf Wirksamkeit bespritzt man junge Reispflanzen im 3- bis 4-Blattstadium mit der Wirkstoffzubereitung bis zur Tropfnässe. Die Pflanzen verbleiben bis zum Abtrocknen im Gewächshaus. Anschließend werden die Pflanzen mit Pellicularia sasakii inokuliert und bei 25°C und einer relativen Luftfeuchtigkeit von 100 % aufgestellt.

5 bis 8 Tage nach der Inokulation erfolgt die Auswertung des Krankheitsbefalles.

Eine deutliche Überlegenheit in der Wirksamkeit gegenüber der Verbindung (B) aus dem Stand der Technik zeigen in diesem Test z.B. die Verbindungen gemäß den Herstellungsbeispielen: 3 und 4.

## Patentansprüche (Patentansprüche für folgende(n) Vertragsstaat(en): BE, CH, DK, ES, FR, GB, IT, LI, NL)

1. Substituierte Pyridylpyrimidine der Formel 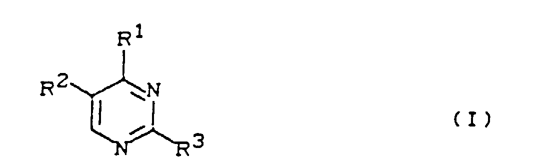 in welcher
R¹ für geradkettiges oder verzweigtes Alkyl mit 1 bis 4 Kohlenstoffatomen, für geradkettiges oder verzweigtes Halogenalkyl mit 1 bis 4-Kohlenstoffatomen und 1 bis 9 gleichen oder verschiedenen Halogenatomen, oder für gegebenenfalls einfach bis vierfach, gleich oder verschieden substituiertes Cycloalkyl mit 3 bis 6 Kohlenstoffatomen steht, wobei als Substituenten infrage kommen:
Halogen, geradkettiges oder verzweigtes Alkyl mit 1 bis 4 Kohlenstoffatomen oder geradkettiges oder verzweigtes Halogenalkyl mit 1 bis 4 Kohlenstoffatomen und 1 bis 9 gleichen oder verschiedenen Halogenatomen,
R² für gegebenenfalls einfach bis fünffach, gleich oder verschieden substituiertes Phenyl steht, wobei als Substituenten jeweils infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Trifluormethyl, Trifluormethoxy, Trifluormethylthio, Methoxycarbonyl, Ethoxycarbonyl, Methoximinomethyl, Methoximinoethyl, Ethoximinomethyl, Ethoximinoethyl oder gegebenenfalls ein- bis dreifach, gleich oder verschieden durch Fluor, Chlor, Brom, Methyl und/oder Ethyl substituiertes Phenyl, und
R³ für gegebenenfalls einfach bis vierfach, gleich oder verschieden substituiertes 2-Pyridyl oder 4-Pyridyl steht, wobei als Substituenten jeweils infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Trifluormethyl, Trifluormethoxy, Trifluormethylthio, Methoxycarbonyl, Ethoxycarbonyl, Methoximinomethyl, Methoximinoethyl, Ethoximinomethyl, Ethoximinoethyl oder gegebenenfalls ein- bis dreifach, gleich oder verschieden durch Fluor, Chlor, Brom, Methyl und/oder Ethyl substituiertes Phenyl.

2. Substituierte Pyridylpyrimidine der Formel (I) gemäß Anspruch 1, in welcher
R¹ Methyl, Ethyl, n- oder i-Propyl, n-, i-, s- oder t-Butyl, für geradkettiges oder verzweigtes Halogenalkyl mit 1 bis 4 Kohlenstoffatomen und 1 bis 5 gleichen oder verschiedenen Halogenatomen oder für jeweils gegebenenfalls einfach oder zweifach, gleich oder verschieden substituiertes Cyclopropyl, Cyclobutyl, Cyclopentyl oder Cyclohexyl steht, wobei als Cycloalkylsubstituenten jeweils infrage kommen: Fluor, Chlor, Brom, Methyl, Ethyl, n- oder i- Propyl, Chlormethyl oder Trifluormethyl,
R² für gegebenenfalls einfach bis dreifach, gleich oder verschieden substituiertes Phenyl steht, wobei als Substituenten jeweils infrage kommen:
Fluor, Chlor, Brom, Cyano, Nitro, Methyl, Ethyl, n- oder i-Propyl, n-, i-, s- oder t- Butyl, Methoxy, Ethoxy, n- oder i-Propoxy, n-, i-, s- oder t-Butoxy, Methylthio, Ethylthio, Trifluormethyl, Trifluormethoxy oder Trifluormethylthio, und
R³ für gegebenenfalls einfach oder zweifach, gleich oder verschieden substituiertes 2-Pyridyl steht, wobei als Substituenten jeweils infrage kommen:
Fluor, Chlor, Brom, Cyano, Nitro, Methyl, Ethyl, n- oder i-Propyl, n-, i-, s- oder t- Butyl, Methoxy, Ethoxy, n- oder i-Propoxy, n-, i-, s- oder t-Butoxy, Methylthio, Ethylthio, Trifluormethyl, Trifluormethoxy oder Trifluormethylthio.

3. Verfahren zur Herstellung von substituierten Pyridylpyrimidinen der Formel (I) gemäß Anspruch 1, dadurch gekennzeichnet, daß man Pyridylamidin Hydrochloride der Formel 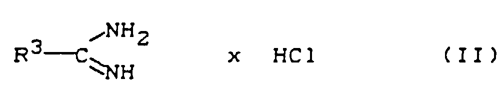 in welcher
R³ die oben angegebene Bedeutung hat,
mit Enaminoketonen der Formel 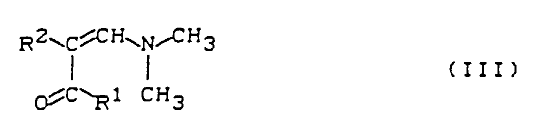 in welcher
R¹ und R² die oben angegebene Bedeutung haben,
gegebenenfalls in Gegenwart eines Verdünnungsmittels und gegebenenfalls in Gegenwart eines Säurebindemittels umsetzt.

4. Mittel zur Bekämpfung phytopathogener Pilze, gekennzeichnet durch einen Gehalt an mindestens einem substituierten Pyridylpyrimidin der Formel (I) gemäß Anspruch 1.

5. Verwendung von substituierten Pyridylpyrimidinen der Formel (I) gemäß Anspruch 1 zur Bekämpfung von phytopathogenen Pilzen.

6. Verfahren zur Bekämpfung von phytopathogenen Pilzen, dadurch gekennzeichnet, daß man substituierte Pyridylpyrimidine der Formel (I) gemäß Anspruch 1 auf die Pilze und/oder deren Lebensraum ausbringt.

7. Verfahren zur Herstellung von Mitteln zur Bekämpfung phytopathogener Pilze, dadurch gekennzeichnet, daß man substituierte Pyridylpyrimidine der Formel (I) gemäß Anspruch 1 mit Streckmitteln und/ oder oberflächenaktiven Stoffen vermischt.

## Patentansprüche (Patentansprüche für folgende(n) Vertragsstaat(en): DE)

1. Substituierte Pyridylpyrimidine der Formel 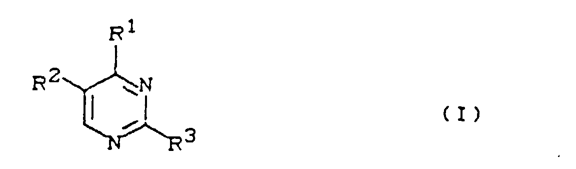 in welcher
R¹ für geradkettiges oder verzweigtes Alkyl mit 1 bis 4 Kohlenstoffatomen, für geradkettiges oder verzweigtes Halogenalkyl mit 1 bis 4 Kohlenstoffatomen und 1 bis 9 gleichen oder verschiedenen Halogenatomen, oder für gegebenenfalls einfach bis vierfach, gleich oder verschieden substituiertes Cycloalkyl mit 3 bis 6 Kohlenstoffatomen steht, wobei als Substituenten infrage kommen:
Halogen, geradkettiges oder verzweigtes Alkyl mit 1 bis 4 Kohlenstoffatomen oder geradkettiges oder verzweigtes Halogenalkyl mit 1 bis 4 Kohlenstoffatomen und 1 bis 9 gleichen oder verschiedenen Halogenatomen,
R² für gegebenenfalls einfach bis fünffach, gleich oder verschieden substituiertes Phenyl steht, wobei als Substituenten jeweils infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Trifluormethyl, Trifluormethoxy, Trifluormethylthio, Methoxycarbonyl, Ethoxycarbonyl, Methoximinomethyl, Methoximinoethyl, Ethoximinomethyl, Ethoximinoethyl oder gegebenenfalls ein- bis dreifach, gleich oder verschieden durch Fluor, Chlor, Brom, Methyl und/oder Ethyl substituiertes Phenyl, und
R³ für gegebenenfalls einfach bis vierfach, gleich oder verschieden substituiertes 2-Pyridyl oder 4-Pyridyl steht, wobei als Substituenten jeweils infrage kommen:
Fluor, Chlor, Brom, Cyano, Nitro, Methyl, Ethyl, n- oder i-Propyl, n-, i-, s- oder t-Butyl, Methylthio, Ethylthio, Trifluormethyl, Trifluormethoxy, Trifluormethylthio, Methoxycarbonyl, Ethoxycarbonyl, Methoximinomethyl, Methoximinoethyl, Ethoximinomethyl, Ethoximinoethyl oder gegebenenfalls ein- bis dreifach, gleich oder verschieden durch Fluor, Chlor, Brom, Methyl und/oder Ethyl substituiertes Phenyl.

2. Substituierte Pyridylpyrimidine der Formel (I) gemäß Anspruch 1, in welcher
R¹ Methyl, Ethyl, n- oder i-Propyl, n-, i-, s- oder t-Butyl, für geradkettiges oder verzweigtes Halogenalkyl mit 1 bis 4 Kohlenstoffatomen und 1 bis 5 gleichen oder verschiedenen Halogenatomen oder für jeweils gegebenenfalls einfach oder zweifach, gleich oder verschieden substituiertes Cyclopropyl, Cyclobutyl, Cyclopentyl oder Cyclohexyl steht, wobei als Cycloalkylsubstituenten jeweils infrage kommen: Fluor, Chlor, Brom, Methyl, Ethyl, n- oder i- Propyl, Chlormethyl oder Trifluormethyl,
R² für gegebenenfalls einfach bis dreifach, gleich oder verschieden substituiertes Phenyl steht, wobei als Substituenten jeweils infrage kommen:
Fluor, Chlor, Brom, Cyano, Nitro, Methyl, Ethyl, n- oder i-Propyl, n-, i-, s- oder t- Butyl, Methoxy, Ethoxy, n- oder i-Propoxy, n-, i-, s- oder t-Butoxy, Methylthio, Ethylthio, Trifluormethyl, Trifluormethoxy oder Trifluormethylthio, und
R³ für gegebenenfalls einfach oder zweifach, gleich oder verschieden substituiertes 2-Pyridyl steht, wobei als Substituenten jeweils infrage kommen:
Fluor, Chlor, Brom, Cyano, Nitro, Methyl, Ethyl, n- oder i-Propyl, n-, i-, s- oder t-Butyl, Methylthio, Ethylthio, Trifluormethyl, Trifluormethoxy oder Trifluormethylthio

3. Verfahren zur Herstellung von substituierten Pyridylpyrimidinen der Formel (I) gemäß Anspruch 1, dadurch gekennzeichnet, daß man Pyridylamidin Hydrochloride der Formel 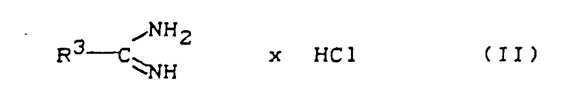 in welcher
R³ die oben angegebene Bedeutung hat,
mit Enaminoketonen der Formel 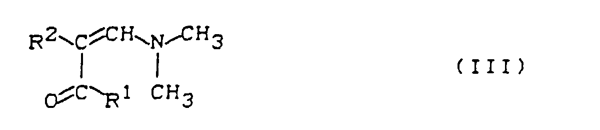 in welcher
R¹ und R² die oben angegebene Bedeutung haben,
gegebenenfalls in Gegenwart eines Verdünnungsmittels und gegebenenfalls in Gegenwart eines Säurebindemittels umsetzt.

4. Mittel zur Bekämpfung phytopathogener Pilze, gekennzeichnet durch einen Gehalt an mindestens einem substituierten Pyridylpyrimidin der Formel (I) gemäß Anspruch 1.

5. Verwendung von substituierten Pyridylpyrimidinen der Formel (I) gemäß Anspruch 1 zur Bekämpfung von phytopathogenen Pilzen.

6. Verfahren zur Bekämpfung von phytopathogenen Pilzen, dadurch gekennzeichnet, daß man substituierte Pyridylpyrimidine der Formel (I) gemäß Anspruch 1 auf die Pilze und/oder deren Lebensraum ausbringt.

7. Verfahren zur Herstellung von Mitteln zur Bekämpfung phytopathogener Pilze, dadurch gekennzeichnet, daß man substituierte Pyridylpyrimidine der Formel (I) gemäß Anspruch 1 mit Streckmitteln und/ oder oberflächenaktiven Stoffen vermischt.

## Claims (Claims for the following Contracting State(s): BE, CH, DK, ES, FR, GB, IT, LI, NL)

1. Substituted pyridylpyrimidines of the formula 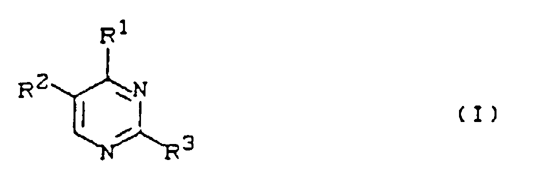 in which
R¹ represents straight-chain or branched alkyl having 1 to 4 carbon atoms, straight-chain or branched halogenoalkyl having 1 to 4 carbon atoms and 1 to 9 identical or different halogen atoms, or represents cycloalkyl which has 3 to 6 carbon atoms and which is optionally monosubstituted to tetrasubstituted by identical or different substituents, suitable substituents being:
halogen, straight-chain or branched alkyl having 1 to 4 carbon atoms or straight-chain or branched halogenoalkyl having 1 to 4 carbon atoms and 1 to 9 identical or different halogen atoms,
R² represents phenyl which is optionally monosubstituted to pentasubstituted by identical or different substituents, suitable substituents in each case being:
fluorine, chlorine, bromine, cyano, nitro, methyl, ethyl, n- or i-propyl, n-, i-, s- or t-butyl, methoxy, ethoxy, n- or i-propoxy, n-, i-, s- or t-butoxy, methylthio, ethylthio, trifluoromethyl, trifluoromethoxy, trifluoromethylthio, methoxycarbonyl, ethoxycarbonyl, methoximinomethyl, methoximinoethyl, ethoximinomethyl, ethoximinoethyl, or phenyl which is optionally monosubstituted to trisubstituted by identical or different substituents from the series comprising fluorine, chlorine, bromine, methyl and/or ethyl, and
R³ represents 2-pyridyl or 4-pyridyl, each of which is optionally monosubstituted to tetrasubstituted by identical or different substituents, suitable substituents in each case being:
fluorine, chlorine, bromine, cyano, nitro, methyl, ethyl, n- or i-propyl, n-, i-, s- or t-butyl, methoxy, ethoxy, n- or i-propoxy, n-, i-, s- or t-butoxy, methylthio, ethylthio, trifluoromethyl, trifluoromethoxy, trifluoromethylthio, methoxycarbonyl, ethoxycarbonyl, methoximinomethyl, methoximinoethyl, ethoximinomethyl, ethoximinoethyl, or phenyl which is optionally monosubstituted to trisubstituted by identical or different substitutents from the series comprising fluorine, chlorine, bromine, methyl and/or ethyl.

2. Substituted pyridylpyrimidines of the formula (I) according to Claim 1, in which
R¹ represents methyl, ethyl, n- or i-propyl, n-, i-, s- or t-butyl, represents straight-chain or branched halogenoalkyl having 1 to 4 carbon atoms and 1 to 5 identical or different halogen atoms, or represents cyclopropyl, cyclobutyl, cyclopentyl or cyclohexyl, each of which is optionally monosubstituted or disubstituted by identical or different substituents, suitable cycloalkyl substituents in each case being:
fluorine, chlorine, bromine, methyl, ethyl, n- or i-propyl, chloromethyl or trifluoromethyl,
R² represents phenyl which is optionally monosubstituted to trisubstituted by identical or different substituents, suitable substituents in each case being:
fluorine, chlorine, bromine, cyano, nitro, methyl, ethyl, n- or i-propyl, n-, i-, s- or t-butyl, methoxy, ethoxy, n- or i-propoxy, n-, i- s- or t-butoxy, methylthio, ethylthio, trifluoromethyl, trifluoromethoxy or trifluoromethylthio, and
R³ represents 2-pyridyl which is optionally monosubstituted or disubstituted by identical or different substituents, suitable substituents in each case being:
fluorine, chlorine, bromine, cyano, nitro, methyl, ethyl, n- or i-propyl, n-, i-, s- or t-butyl, methoxy, ethoxy, n- or i-propoxy, n-, i-, s- or t-butoxy, methylthio, ethylthio, trifluoromethyl, trifluoromethoxy or trifluoromethylthio.

3. Process for the preparation of substituted pyrimidines of the formula (I), according to Claim 1,
characterized in that
pyridylamidine hydrochlorides of the formula 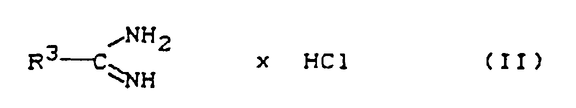 in which
R³ has the abovementioned meaning,
are reacted with enamino ketones of the formula 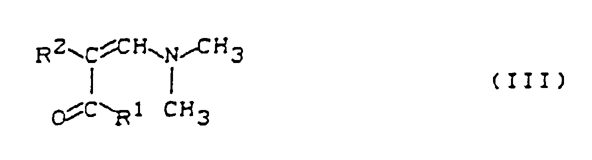 in which
R¹ and R² have the abovementioned meaning,
if appropriate in the presence of a diluent and if appropriate in the presence of an acid-binding agent.

4. Compositions for controlling phytopathogenic fungi, characterized in that they comprise at least one substituted pyridylpyrimidine of the formula (I) according to Claim 1.

5. Use of substituted pyridylpyrimidines of the formula (I) according to Claim 1 for controlling phytopathogenic fungi.

6. Method for controlling phytopathogenic fungi, characterized in that substituted pyridylpyrimidines of the formula (I) according to Claim 1 are applied to the fungi and/or their habitat.

7. Process for preparing compositions for controlling phytopathogenic fungi, characterized in that substituted pyridylpyrimidines of the formula (I) according to Claim 1 are mixed with extenders and/or surfactants.

## Claims (Claims for the following Contracting State(s): DE)

1. Substituted pyridylpyrimidines of the formula 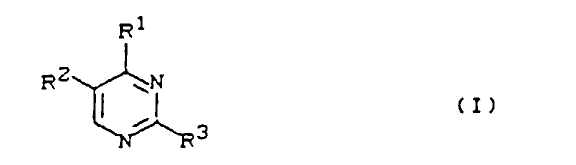 in which
R¹ represents straight-chain or branched alkyl having 1 to 4 carbon atoms, straight-chain or branched halogenoalkyl having 1 to 4 carbon atoms and 1 to 9 identical or different halogen atoms, or represents cycloalkyl which has 3 to 6 carbon atoms and which is optionally monosubstituted to tetrasubstituted by identical or different substituents, suitable substituents being:
halogen, straight-chain or branched alkyl having 1 to 4 carbon atoms or straight-chain or branched halogenoalkyl having 1 to 4 carbon atoms and 1 to 9 identical or different halogen atoms,
R² represents phenyl which is optionally monosubstituted to pentasubstituted by identical or different substituents, suitable substituents in each case being:
fluorine, chlorine, bromine, cyano, nitro, methyl, ethyl, n- or i-propyl, n-, i-, s- or t-butyl, methoxy, ethoxy, n- or i-propoxy, n-, i-, s- or t-butoxy, methylthio, ethylthio, trifluoromethyl, trifluoromethoxy, trifluoromethylthio, methoxycarbonyl, ethoxycarbonyl, methoximinomethyl, methoximinoethyl, ethoximinomethyl, ethoximinoethyl, or phenyl which is optionally monosubstituted to trisubstituted by identical or different substituents from the series comprising fluorine, chlorine, bromine, methyl and/or ethyl, and
R³ represents 2-pyridyl or 4-pyridyl, each of which is optionally monosubstituted to tetrasubstituted by identical or different substituents, suitable substituents in each case being:
fluorine, chlorine, bromine, cyano, nitro, methyl, ethyl, n- or i-propyl, n-, i-, s- or t-butyl, methylthio, ethylthio, trifluoromethyl, trifluoromethoxy, trifluoromethylthio, methoxycarbonyl, ethoxycarbonyl, methoximinomethyl, methyoximinoethyl, ethoximinomethyl, ethoximinoethyl, or phenyl which is optionally monosubstituted to trisubstituted by identical or different substituents from the series comprising fluorine, chlorine, bromine, methyl and/or ethyl.

2. Substituted pyridylpyrimidines of the formula (I) according to Claim 1, in which
R¹ represents methyl, ethyl, n- or i-propyl, n-, i-, s- or t-butyl, represents straight-chain or branched halogenoalkyl having 1 to 4 carbon atoms and 1 to 5 identical or different halogen atoms, or represents cyclopropyl, cyclobutyl, cyclopentyl or cyclohexyl, each of which is optionally monosubstituted or disubstituted by identical or different substituents, suitable cycloalkyl substituents in each case being:
fluorine, chlorine, bromine, methyl, ethyl, n- or i-propyl, chloromethyl or trifluoromethyl,
R² represents phenyl which is optionally monosubstituted to trisubstituted by identical or different substituents, suitable substituents in each case being:
fluorine, chlorine, bromine, cyano, nitro, methyl, ethyl, n- or i-propyl, n-, i-, s- or t-butyl, methoxy, ethoxy, n- or i-propoxy, n-, i-, s- or t-butoxy, methylthio, ethylthio, trifluoromethyl, trifluoromethoxy or trifluoromethylthio, and
R³ represents 2-pyridyl which is optionally monosubstituted or disubstituted by identical or different substituents, suitable substituents in each case being:
fluorine, chlorine, bromine, cyano, nitro, methyl, ethyl, n- or i-propyl, n-, i-, s- or t-butyl, methylthio, ethylthio, trifluromethyl, trifluromethoxy or trifluromethylthio.

3. Process for the preparation of substituted pyrimidines of the formula (I) according to Claim 1,
characterized in that
pyridylamidine hydrochlorides of the formula 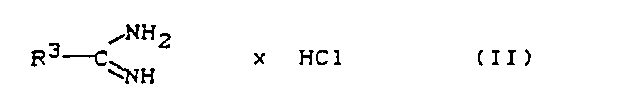 in which
R³ has the abovementioned meaning,
are reacted with enamino ketones of the formula 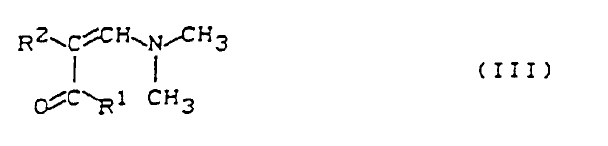 in which
R¹ and R² have the abovementioned meaning,
if appropriate in the presence of a diluent and if appropriate in the presence of an acid-binding agent.

4. Compositions for controlling phytopathogenic fungi, characterized in that they comprise at least one substituted pyridylpyrimidine of the formula (I) according to Claim 1.

5. Use of substituted pyridylpyrimidines of the formula (I) according to Claim 1 for controlling phytopathogenic fungi.

6. Method for controlling phytopathogenic fungi, characterized in that substituted pyridylpyrimidines of the formula (I) according to Claim 1 are applied to the fungi and/or their habitat.

7. Process for preparing compositions for controlling phytopathogenic fungi, characterized in that substituted pyridylpyrimidines of the formula (I) according to Claim 1 are mixed with extenders and/or surfactants.

## Revendications (Revendications pour l'(les) Etat(s) contractant(s) suivant(s): BE, CH, DK, ES, FR, GB, IT, LI, NL)

1. Pyridylpyrimidines substituées de formule 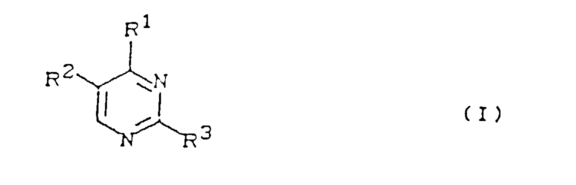 dans laquelle
R¹ est un groupe alkyle linéaire ou ramifié ayant 1 à 4 atomes de carbone, un groupe halogénalkyle linéaire ou ramifié ayant 1 à 4 atomes de carbone et 1 à 9 atomes d'halogènes identiques ou différents ou un groupe cycloalkyle de 3 à 6 atomes de carbone éventuellement substitué une à quatre fois identiques ou différentes, les substituants considérés étant :
un halogène, un radical alkyle linéaire ou ramifié ayant 1 à 4 atomes de carbone ou un radical halogénalkyle linéaire ou ramifié ayant 1 à 4 atomes de carbone et 1 à 9 atomes d'halogènes identiques ou différents,
R² est un groupe phényle portant éventuellement 1 à 5 substituants identiques ou différents, les substituants considérés étant dans chaque cas :
le fluor, le chlore, le brome, un radical cyano, nitro, méthyle, éthyle, n-propyle, isopropyle, n-butyle, iso-butyle, sec.-butyle, tertio-butyle, méthoxy, éthoxy, n-propoxy, isopropoxy, n-butoxy, iso-butoxy, sec.-butoxy, tertio-butoxy, méthylthio, éthylthio, trifluorométhyle, trifluorométhoxy, trifluorométhylthio, méthoxycarbonyle, éthoxy-carbonyle, méthoximinométhyle, méthoximinoéthyle, éthoximinométhyle, éthoximinoéthyle ou phényle éventuellement substitué une à trois fois identiques ou différentes par du fluor, du chlore, du brome, un radical méthyle et/ou éthyle, et
R³ est un groupe 2-pyridyle ou 4-pyridyle portant éventuellement 1 à 4 substituants identiques ou différents, les substituants considérés étant dans chaque cas :
le fluor, le chlore, le brome, un radical cyano, nitro, méthyle, éthyle, n-propyle, isopropyle, n-butyle, iso-butyle, sec.-butyle, tertio-butyle, méthoxy, éthoxy, n-propoxy, isopropoxy, n-butoxy, iso-butoxy, sec.-butoxy, tertio-butoxy, méthylthio, éthylthio, trifluorométhyle, trifluorométhoxy, trifluorométhylthio, méthoxycarbonyle, éthoxycarbonyle, méthoximinométhyle, méthoximinoéthyle, éthoximinométhyle, éthoximinoéthyle ou phényle portant le cas échéant 1 à 3 substituants, identiques ou différents, fluor, chlore, brome, méthyle et/ou éthyle.

2. Pyridylpyrimidines substituées de formule (I) suivant la revendication 1, dans laquelle
R¹ est un groupe méthyle, éthyle, n-propyle, iso-propyle, n-butyle, iso-butyle, sec.-butyle ou tertio-butyle, un groupe halogénalkyle linéaire ou ramifié ayant 1 à 4 atomes de carbone et 1 à 5 atomes d'halogènes identiques ou différents ou un groupe cyclopropyle, cyclobutyle, cyclopentyle ou cyclohexyle portant éventuellement dans chaque cas 1 ou 2 substituants identiques ou différents, les substituants considérés des groupes cycloalkyle étant dans chaque cas : le fluor, le chlore, le brome, un radical méthyle, éthyle, n-propyle, isopropyle, chlorométhyle ou trifluorométhyle,
R² est un groupe phényle portant éventuellement 1 à 3 substituants identiques ou différents, les substituants considérés étant dans chaque cas :
le fluor, le chlore, le brome, un radical cyano, nitro, méthyle, éthyle, n-propyle, isopropyle, n-butyle, iso-butyle, sec.-butyle, tertio-butyle, méthoxy, éthoxy, n-propoxy, isopropoxy, n-butoxy, iso-butoxy, sec.-butoxy, tertio-butoxy, méthylthio, éthylthio, trifluorométhyle, trifluorométhoxy ou trifluorométhylthio, et
R³ est un groupe 2-pyridyle portant éventuellement 1 ou 2 substituants identiques ou différents, les substituants considérés étant dans chaque cas les suivants :
fluor, chlore, brome, cyano, nitro, méthyle, éthyle, n-propyle, isopropyle, n-butyle, iso-butyle, sec.-butyle, tertio-butyle, méthoxy, éthoxy, n-propoxy, isopropoxy, n-butoxy, iso-butoxy, sec.-butoxy, tertio-butoxy, méthylthio, éthylthio, trifluorométhyle, trifluorométhoxy ou trifluorométhylthio.

3. Procédé de production de pyridylpyrimidines substituées de formule (I) suivant la revendication 1, caractérisé en ce qu'on fait réagir des chlorhydrates de pyridylamidines de formule 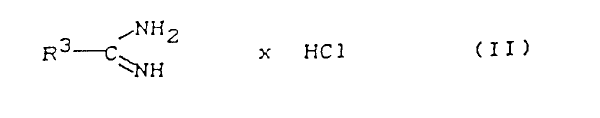 dans laquelle
R³ a la définition indiquée ci-dessus,
avec des énaminocétones de formule 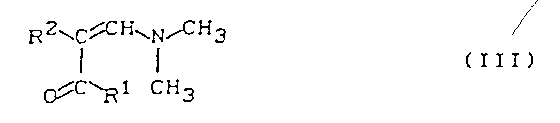 dans laquelle
R¹ et R² ont la définition indiquée ci-dessus,
le cas échéant en présence d'un diluant et en présence éventuelle d'un accepteur d'acide.

4. Compositions destinées à la lutte contre des champignons phytopathogènes, caractérisées par une teneur en au moins une pyridylpyrimidine substituée de formule (I) suivant la revendication 1.

5. Utilisation de pyridylpyrimidines substituées de formule (I) suivant la revendication 1 pour combattre des champignons phytopathogènes.

6. Procédé pour combattre des champignons phytopathogènes, caractérisé en ce qu'on épand des pyridylpyrimidines substituées de formule (I) suivant la revendication 1 sur les champignons et/ou sur leur milieu.

7. Procédé de préparation de compositions destinées à combattre des champignons phytopathogènes, caractérisé en ce qu'on mélange des pyridylpyrimidines substituées de formule (I) suivant la revendication 1 avec des diluants et/ou des agents tensio-actifs.

## Revendications (Revendications pour l'(les) Etat(s) contractant(s) suivant(s): DE)

1. Pyridylpyrimidines substituées de formule 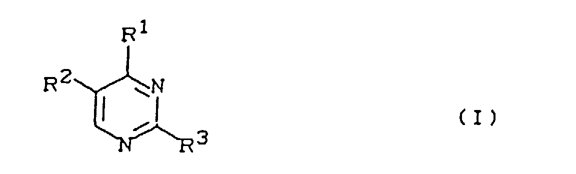 dans laquelle
R¹ est un groupe alkyle linéaire ou ramifié ayant 1 à 4 atomes de carbone, un groupe halogénalkyle linéaire ou ramifié ayant 1 à 4 atomes de carbone et 1 à 9 atomes d'halogènes identiques ou différents ou un groupe cycloalkyle de 3 à 6 atomes de carbone éventuellement substitué une à quatre fois identiques ou différentes, les substituants considérés étant :
un halogène, un radical alkyle linéaire ou ramifié ayant 1 à 4 atomes de carbone ou un radical halogénalkyle linéaire ou ramifié ayant 1 à 4 atomes de carbone et 1 à 9 atomes d'halogènes identiques ou différents,
R² est un groupe phényle portant éventuellement 1 à 5 substituants identiques ou différents, Tes substituants considérés étant dans chaque cas :
le fluor, le chlore, le brome, un radical cyano, nitro, méthyle, éthyle, n-propyle, isopropyle, n-butyle, iso-butyle, sec.-butyle, tertio-butyle, méthoxy, éthoxy, n-propoxy, isopropoxy, n-butoxy, iso-butoxy, sec.-butoxy, tertio-butoxy, méthylthio, éthylthio, trifluorométhyle, trifluorométhoxy, trifluorométhylthio, méthoxycarbonyle, éthoxycarbonyle, méthoximinométhyle, méthoximino-éthyle, éthoximinométhyle, éthoximino-éthyle ou phényle éventuellement substitué une à trois fois identiques ou différentes par du fluor, du chlore, du brome, un radical méthyle et/ou éthyle, et
R³ est un groupe 2-pyridyle ou 4-pyridyle portant éventuellement 1 à 4 substituants identiques ou différents, les substituants considérés étant dans chaque cas :
le fluor, le chlore, le brome, un radical cyano, nitro, méthyle, éthyle, n-propyle, isopropyle, n-butyle, iso-butyle, sec.-butyle, tertio-butyle, méthylthio, éthylthio, trifluorométhyle, trifluorométhoxy, trifluorométhylthio, méthoxycarbonyle, éthoxycarbonyle, méthoximinométhyle, méthoximinoéthyle, éthoximinométhyle, éthoximino-éthyle ou phényle portant le cas échéant 1 à 3 substituants, identiques ou différents, fluor, chlore, brome, méthyle et/ou éthyle.

2. Pyridylpyrimidines substituées de formule (I) suivant la revendication 1, dans laquelle
R¹ est un groupe méthyle, éthyle, n-propyle, iso-propyle, n-butyle, iso-butyle, sec.-butyle ou tertio-butyle, un groupe halogénalkyle linéaire ou ramifié ayant 1 à 4 atomes de carbone et 1 à 5 atomes d'halogènes identiques ou différents ou un groupe cyclopropyle, cyclobutyle, cyclopentyle ou cyclohexyle portant éventuellement dans chaque cas 1 ou 2 substituants identiques ou différents, les substituants considérés des groupes cycloalkyle étant dans chaque cas : le fluor, le chlore, le brome, un radical méthyle, éthyle, n-propyle, isopropyle, chlorométhyle ou trifluorométhyle,
R² est un groupe phényle portant éventuellement 1 à 3 substituants identiques ou différents, les substituants considérés étant dans chaque cas :
le fluor, le chlore, le brome, un radical cyano, nitro, méthyle, éthyle, n-propyle, isopropyle, n-butyle, iso-butyle, sec.-butyle, tertio-butyle, méthoxy, éthoxy, n-propoxy, isopropoxy, n-butoxy, iso-butoxy, sec.-butoxy, tertio-butoxy, méthylthio, éthylthio, trifluorométhyle, trifluorométhoxy ou trifluorométhylthio, et
R³ est un groupe 2-pyridyle portant éventuellement 1 ou 2 substituants identiques ou différents, les substituants considérés étant dans chaque cas les suivants :
fluor, chlore, brome, cyano, nitro, méthyle, éthyle, n-propyle, isopropyle, n-butyle, iso-butyle, sec.-butyle, tertio-butyle, méthylthio, éthylthio, trifluorométhyle, trifluorométhoxy ou trifluorométhylthio.

3. Procédé de production de pyridylpyrimidines substituées de formule (I) suivant la revendication 1, caractérisé en ce qu'on fait réagir des chlorhydrates de pyridylamidines de formule 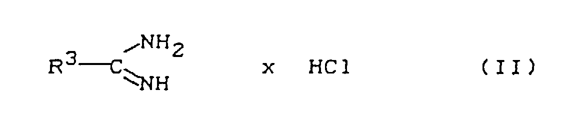 dans laquelle
R³ a la définition indiquée ci-dessus,
avec des énaminocétones de formule 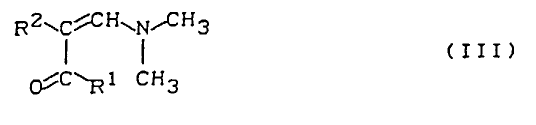 dans laquelle
R¹ et R² ont la définition indiquée ci-dessus,
le cas échéant en présence d'un diluant et en présence éventuelle d'un accepteur d'acide.

4. Compositions destinées à la lutte contre des champignons phytopathogènes, caractérisées par une teneur en au moins une pyridylpyrimidine substituée de formule (I) suivant la revendication 1.

5. Utilisation de pyridylpyrimidines substituées de formule (I) suivant la revendication 1 pour combattre des champignons phytopathogènes.

6. Procédé pour combattre des champignons phytopathogènes, caractérisé en ce qu'on épand des pyridylpyrimidines substituées de formule (I) suivant la revendication 1 sur les champignons et/ou sur leur milieu.

7. Procédé de préparation de compositions destinées à combattre des champignons phytopathogènes, caractérisé en ce qu'on mélange des pyridylpyrimidines substituées de formule (I) suivant la revendication 1 avec des diluants et/ou des agents tensio-actifs.
